# EUROPEAN PATENT SPECIFICATION

(11) **EP 2 297 217 B1**
(45) Date of publication and mention of the grant of the patent: **20.10.2021**
(21) Application number: 09795084.4
(22) Date of filing: 07.07.2009
(51) Int. Cl.: C08F 283/04, C08F 283/00, C08F 283/02, C08F 283/06, C08F 297/04, C08L 75/16, C08L 75/06, C08L 75/04, C08F 220/06, C08F 222/10, C08G 18/83, C08L 33/02

(54) **HYDROPHILIC INTERPENETRATING POLYMER NETWORKS DERIVED FROM HYDROPHOBIC POLYMERS**
AUS HYDROPHOBEN POLYMEREN ABGELEITETE NETZWERKE AUS INTERPENETRIERENDEN HYDROPHILEN POLYMEREN
ALLIAGE IPN HYDROPHILE ISSU DE POLYMÈRES HYDROPHOBES

(30) Priority: 07.07.2008 US 78741 P; 08.07.2008 US 79060 P; 08.09.2008 US 95273 P; 02.04.2009 US 166194 P
(43) Date of publication of application: 23.03.2011
(73) Proprietor: Hyalex Orthopaedics, Inc., Lexington, MA 02421 (US)
(72) Inventor: MYUNG, David, Santa Clara, CA (US); JAASMA, Michael J., San Francisco, CA (US); KOURTIS, Lampros, Cambridge, MA (US); FRANK, Curt, Cupertino, CA (US)
(74) Representative: J A Kemp LLP
(86) International application number: PCT/US2009/049846
(87) International publication number: WO 2010/005992

(56) References cited:
- EP-A2- 2 268 331
- US-A- 5 094 876
- US-A- 5 856 366
- US-A- 6 140 452
- US-A1- 2004 266 941
- US-A1- 2005 090 612
- US-A1- 2008 070 086
- US-B1- 6 331 578
- YANG J-M ET AL: "Preparation of poly(acrylic acid) modified polyurethane membrane for biomaterial by UV radiation without degassing", JOURNAL OF BIOMEDICAL MATERIALS RESEARCH, WILEY, NEW YORK, NY, US, vol. 45, no. 2, 1 May 1999 (1999-05-01), pages 133-139, XP002621051, ISSN: 0021-9304
- HSIEH K H ET AL: "COMPATIBILITY AND MORPHOLOGY IN POLYURETHANE AND POLYSTYRENE IONOMERIC INTERPENETRATING POLYMER NETWORKS", POLYMER JOURNAL, SOCIETY OF POLYMER SCIENCE, TOKYO, JP, vol. 21, no. 1, 15 January 1989 (1989-01-15), pages 1-10, XP000126132, ISSN: 0032-3896, DOI: 10.1295/POLYMJ.21.1
- None

## Description

### CROSS REFERENCE TO RELATED APPLICATIONS

This application claims the benefit of U.S. Patent Appl. No. 61/078,741, filed July 7, 2008; U.S. Patent Appl. No. 61/079,060, filed July 8, 2008; U.S. Patent Appl. No. 61/095,273, filed September 8, 2008; and U.S. Patent Appl. No. 61/166,194, filed April 2, 2009.

### FIELD OF THE INVENTION

The present invention pertains to semi- and fully interpenetrating polymer networks.

### BACKGROUND OF THE INVENTION

Fully interpenetrating polymer networks (IPN's) and semi-interpenetrating polymer networks ("semi-IPN's") have been created from a variety of starting materials and have been used for a variety of applications. IPN's and semi-IPN's can combine the beneficial properties of the polymers from which they are made and can avoid some of the undesirable properties of their component polymers.

Prior IPN's and semi-IPN's have been proposed for use in biomedical applications, such as a coating for an implant or as artificial cartilage. See, e.g., U.S. Patent Publ. No. 2005/0147685; U.S. Patent Publ. No. 2009/0035344; and U.S. Patent Publ. No. 2009/008846. The utility of prior IPN's and semi-IPN's for their proposed applications is limited by the properties of those compositions, however. In addition, the starting materials and processes of making such prior compositions limit not only the resulting properties of the IPN or semi-IPN but also the commercial viability of the manufacturing processes and the articles made in such processes. Also, the mechanical properties of prior IPNs and semi-IPNs are often limited by the mechanical properties of the component polymers used, which in the case of most intrinsically hydrophilic, water-swellable polymers, are usually quite low. For example, the prior art has not described a viable process for making a water-swellable IPN or semi-IPN from commercially available hydrophobic thermoset or thermoplastic polymers, such as polyurethane or ABS.

Finally, the utility of prior IPN and semi-IPN compositions and the value of the articles formed from such compositions have been limited by the inability to create IPN's and semi-IPN's with desired characteristics, such as strength, lubricity and wear-resistance.

US 6,331,578 describes a method of making an IPN of at least a first polymer and a second polymer from a first and second component wherein the first component is the first polymer, or a polymerizable reactant selected from monomers or pre-polymers polymerizable to the first polymer and mixtures thereof, and the second component is a polymerizable reactant selected from monomers or pre-polymers polymerizable to the second polymer and mixtures thereof, the method comprising the steps of forming a reaction mixture of at least the first component, the second component, an initiator or catalyst and a crosslinker, effecting polymerization and crosslinking of polymerizable reactant, and recovering the IPN so obtained, wherein during the polymerization and crosslinking, a surface of the reaction mixture is maintained in contact with a third component comprising each said polymerizable reactant at a chemical potential similar to the chemical potential of the same reactant in the reaction mixture.

Yang, J.-M. et al., J. Biomed. Mater. Res., 45(2), 133-139, describes the preparation of poly(acrylic acid) modified polyurethane membranes for biomaterial by UV radiation without degassing.

Hsieh, K. H. et al., Polym. J., 21(1), 1-10 (1989), describes the preparation of IPNs, composed of polyurethane (PU) and poly(styrene-acrylic acid) (PSAA), containing mutual opposite charge groups, i.e., tertiary amine group in PU and carboxyl groups in PSAA, in PU/PSAA ratio of 75/25 (PU-dominated), and 25/75 (PSAA-dominated).

EP-A-2268331 describes a method of attaching an implant to a bone, the implant comprising a hydrated polymer comprising a lubricious hydrated surface and an attachment surface comprising accessible chemical functional groups. The method includes the steps of treating the implant or the bone with an isocyanate-containing compound; placing the attachment surface in apposition to the bone; and allowing the isocyanate-containing compound to cure to bond the implant to the bone. Also described is a medical implant having a hydrated polymer comprising an attachment surface comprising a thermoplastic material, the hydrated polymer having an IPN with at least two polymers, the hydrated polymer having a low coefficient of friction on at least one surface.

US 2004/266941 describes a process for the production of polymeric moldings, such as medical device moldings and optical and ophthalmic lenses, preferably contact lenses and intraocular lenses. Also described are a polymeric precursor mixture said to be useful in polymeric moldings and methods of making and using the polymeric precursor mixture. The semi-solid polymerizable precursor mixture comprises (i) a polymer blend, wherein the polymer blend consists of at least two dissimilar prepolymers or at least one prepolymer and a dead polymer; (ii) at least one non-reactive diluent; and (iii) optionally, at least one reactive plasticizer.

US 2005/090612 describes the preparation of a molded component or article for biomedical use from a crosslinkable non-water-soluble polymer which when crosslinked and saturated with water forms a hydrogel. The polymer is formulated as a composition containing a non-aqueous diluent in addition to the polymer, the diluent being present in a volumetric proportion that is substantially equal to the volumetric proportion of water in the hydrogel that would be formed when the polymer is crosslinked and saturated with water. The composition is cast in a mold where the composition is exposed to conditions that cause crosslinking to occur by a reaction to which the non-aqueous diluent is inert. The crosslinking reaction produces a molded non-aqueous gel which is then converted to a hydrogel by substituting an aqueous liquid such as water or physiological saline for the non-aqueous diluent. The use of a molding composition whose curing consists essentially entirely of crosslinking results in a molding process that is said to entail little or no shrinkage, and dimensional integrity is said to be maintained up through the formation of the hydrogel by using the non-aqueous diluent in essentially the same volumetric proportion as water in the hydrogel.

US 6,140,452 describes a method and related composition and apparatus for repairing a tissue site. The method involves the use of a curable polyurethane biomaterial composition having a plurality of parts adapted to be mixed at the time of use in order to provide a flowable composition and to initiate cure. The flowable composition can be delivered using minimally invasive means to a tissue site and there fully cured provide a permanent and biocompatible prosthesis for repair of the tissue site. Further provided are a mold apparatus, e.g., in the form of a balloon or tubular cavity, for receiving a biomaterial composition, and a method for delivering and filling the mold apparatus with a curable composition in situ to provide a prosthesis for tissue repair.

US 2008/070086 describes a phase-separated polymer electrolyte membrane which is said to have a low degree of swelling in water and methanol, to excel in resistance to water and methanol, and to have high methanol barrier property and a low membrane resistance. An electrode-phase-separated polymer electrolyte membrane joint using the membrane, a method for manufacturing the membrane and joint, and a fuel cell using them are also provided. There is provided a polymer electrolyte membrane having two phases: a domain phase comprising an electrolyte polymer (a) and a matrix phase comprising a polymer (b) that inhibits swelling of component (a), the membrane also having a three-dimensional structure that can conduct protons and substantially connects the domains of (a).

US 5,856,366 describes a High Internal Phase Emulsion (HIPE)-derived heterogeneous polymeric foam structure of interconnected open-cells, wherein the foam structure has at least two distinct regions. Such heterogeneous foams are said to have various applications, such as energy and fluid absorption, insulation, and filtration. Also described is a heterogeneous absorbent polymeric foam that, upon contact with aqueous fluids (in particular body fluids such as urine and blood), is said to be able to acquire, distribute, and store these fluids. The foams have at least two distinct regions having different density, polymer composition, surface properties, and/or microcellular morphology. Also described is a process for obtaining the heterogeneous foams by polymerizing a high internal phase water-in-oil emulsion, or HIPE. In one aspect, the process utilizes at least two distinct HIPEs, with each emulsion having a relatively small amount of an oil phase and a relatively greater amount of a water phase.

### SUMMARY OF THE INVENTION

The mechanical properties desired for certain medical applications is often outside the range of possibility of many hydrophilic starting materials. Hence, one aspect of this invention takes advantage of the high mechanical strength of hydrophobic starting materials and combines those materials with certain ionic polymers as a useful way to achieve the goal of high mechanical strength in addition to other desirable properties. Thus, while the prior art took water-swellable polymers and tried to make them stronger, one aspect of this invention takes strong materials and makes them more water-swellable.

For purposes of this application, an "interpenetrating polymer network" or "IPN" is a material comprising two or more polymer networks which are at least partially interlaced on a molecular scale, but not covalently bonded to each other, and cannot be separated unless chemical bonds are broken. A "semi-interpenetrating polymer network" or "semi-IPN" is a material comprising one or more polymer networks and one or more linear or branched polymers characterized by the penetration on a molecular scale of at least one of the networks by at least some of the linear or branched macromolecules. As distinguished from an IPN, a semi-IPN is a polymer blend in which at least one of the component polymer networks is not chemically crosslinked by covalent bonds.

A "polymer" is a substance comprising macromolecules, including homopolymers (a polymer derived one species of monomer) and copolymers (a polymer derived from more than one species of monomer). A "hydrophobic polymer" is a pre-formed polymer network having at least one of the following two properties: (1) a surface water contact angle of at least 45° and (2) exhibits water absorption of 2.5% or less after 24 hours at room temperature according to ASTM test standard D570. A "hydrophilic polymer" is a polymer network having a surface water contact angle less than 45° and exhibits water absorption of more than 2.5% after 24 hours at room temperature according to ASTM test standard D570. An "ionic polymer" is defined as a polymer comprised of macromolecules containing at least 2% by weight ionic or ionizable monomers (or both), irrespective of their nature and location. An "ionizable monomer" is a small molecule that can be chemically bonded to other monomers to form a polymer and which also has the ability to become negatively charged due the presence of acid functional groups such carboxylic acid and/or sulfonic acid. A "thermoset polymer" is one that doesn't melt when heated, unlike a thermoplastic polymer. Thermoset polymers "set" into a given shape when first made and afterwards do not flow or melt, but rather decompose upon heating and are often highly crosslinked and/or covalently crosslinked. A "thermoplastic polymer" is one which melts or flows when heated, unlike thermoset polymers. Thermoplastic polymers are usually not covalently crosslinked. "Phase separation" is defined as the conversion of a single-phase system into a multi-phase system; especially the separation of two immiscible blocks of a block co-polymer into two phases, with the possibility of a small interphase in which a small degree of mixing occurs. The present disclosure includes a process for modifying common commercially available hydrophobic thermoset or thermoplastic polymers, such as polyurethane or ABS to provide new properties, such as strength, lubricity, electrical conductivity and wear-resistance. Other possible hydrophobic thermoset or thermoplastic polymers are described below. The disclosure also includes the IPN and semi-IPN compositions as well as articles made from such compositions and methods of using such articles. The IPN and semi-IPN compositions may attain one or more of the following characteristics: High tensile and compressive strength; low coefficient of friction; high water content and swellability; high permeability; biocompatibility; and biostability.

Applications of the invention are the creation of hydrophilic, lubricious sidings or coatings to reduce the static and dynamic coefficient of friction between two bearing surfaces and to reduce drag and/or biofilm formation and/or barnacle formation in marine vessels, diving or swimming suits, other water crafts or water-borne objects, or pipes. Furthermore, the invention has potential in electrochemical applications that require conduction of electrical current, or permeability of ions such as proton exchange membranes, fuel cells, filtration devices, and ion-exchange membranes. In addition, the invention can be used as a method for making bearings and moving parts for applications such as engines, pistons, or other machines or machine parts. The invention can also be used in numerous biomedical applications including cartilage substitutes, orthopaedic joint replacement and resurfacing devices or components thereof, intervertebral discs, stents, vascular or urinary catheters, condoms, heart valves, vascular grafts, and both short-term and long-term implants in other areas of the body, such as skin, brain, spine, the gastro-intestinal system, the larynx, and soft tissues in general. In addition, it can be used as a component of various surgical tools and instruments. In all of these applications drugs can be incorporated into the material for localized drug delivery. These interpenetrating polymer networks can also be used to fabricate specific drug delivery vehicles in which a therapeutic agent is released from the polymer matrix.

The present invention accordingly provides a composition of matter comprising a negatively charged water-swellable IPN or semi-IPN comprising a hydrophobic thermoset or thermoplastic polyurethane polymer and an ionic polymer comprising sulfonate groups physically entangled with the polyurethane, wherein the ionic polymer forms a concentration gradient from a first portion of the composition to a second portion of the composition.

In some embodiments, the IPN or semi-IPN exhibits a lower coefficient of friction than the hydrophobic thermoset or thermoplastic polymer. In some embodiments, the IPN or semi-IPN is more water-swellable, exhibits higher resistance to creep, and/or exhibits a higher conductivity and permeability than the hydrophobic thermoset or thermoplastic polymer. Some embodiments of the composition also include an anti-oxidation agent.

In some embodiments, the IPN or semi-IPN is formed by diffusing an ionizable monomer precursor solution into the hydrophobic thermoset or thermoplastic polymer and polymerizing the monomers to form the ionic polymer.

In some embodiments, the composition also includes water, which may form a hydration gradient from a first portion of the composition to a second portion of the composition. An electrolyte may be dissolved in the water. In various embodiments, the hydrophobic thermoset or thermoplastic polymer may be chemically crosslinked with the ionic polymer.

In some embodiments, the hydrophobic thermoset or thermoplastic polymer has ordered and disordered domains, and the ionic polymer may be disposed in the disordered domains.

The ionic polymer may be, e.g., a poly(sulfopropyl methacrylate), combinations of a poly(acrylic acid) and poly(sulfopropyl methacrylate), or derivatives thereof. The ionic polymer may include carboxylate groups and sulfonate groups.

The concentration gradient formed by the ionic polymer from a first portion of the composition to a second portion of the composition may, e.g., provide a stiffness and/or hydration gradient within the composition.

Some embodiments include a second hydrophobic thermoset or thermoplastic polymer which may be disposed in a layer separate from the first hydrophobic thermoset or thermoplastic polymer or may be diffused throughout the first hydrophobic thermoset or thermoplastic polymer.

The present disclosure includes a process for producing a water-swellable IPN or semi-IPN from an hydrophobic thermoset or thermoplastic polymer including the following steps: placing an ionizable monomer solution in contact with a solid form of the hydrophobic thermoset or thermoplastic polymer; diffusing the ionizable monomer solution into the thermoset or thermoplastic polymer; and polymerizing the ionizable monomers to form a ionic polymer inside the thermoset or thermoplastic polymer, thereby forming the IPN or semi-IPN.

Some embodiments include the step of adding an anti-oxidation agent. Some embodiments include the step of swelling the IPN or semi-IPN with water, e.g., to form a hydration gradient from a first portion of the composition to a second portion of the composition. The method may also include the step of swelling the IPN or semi-IPN with an electrolyte solution. Various embodiments include the steps of chemically crosslinking or physically entangling the hydrophobic thermoset or thermoplastic polymer with the ionic polymer.

In embodiments in which the hydrophobic thermoset or thermoplastic polymer has ordered and disordered domains, the method may include the step of swelling the disordered domains with the ionizable monomer solution prior to the polymerizing step.

In some embodiments, the hydrophobic thermoset or thermoplastic polymer is selected from the group consisting of polyurethane, polymethyl methacrylate, polydimethylsiloxane, and acrylonitrile butadiene styrene. The ionizable monomer solution may be an acrylic acid solution and may comprise monomers with carboxylate groups and/or sulfonate groups.

In some embodiments, the method includes the step of forming a concentration gradient of the ionic polymer within the IPN or semi-IPN through regioselective diffusion of the ionizable monomer solution through the hydrophobic thermoset of thermoplastic polymer to, e.g., provide a stiffness and/or hydration gradient within the composition.

Some embodiments of the method may include, prior to the polymerizing step, the steps of placing the ionizable monomer solution in contact with a solid form of a second hydrophobic thermoset or thermoplastic polymer; and diffusing the ionizable monomer solution into the second hydrophobic thermoset or thermoplastic polymer. In such embodiments, the second hydrophobic thermoset or thermoplastic polymer may be in a separate layer adjacent to the first hydrophobic thermoset or thermoplastic polymer or may be diffused within the first hydrophobic thermoset or thermoplastic polymer.

Some embodiments include the step of changing the IPN or semi-IPN from a first shape to a second shape, such as by heating the IPN or semi-IPN.

The present disclosure also includes a medical implant including a water-swellable IPN or semi-IPN including an hydrophobic thermoset or thermoplastic polymer and an ionic polymer, the implant having a bone contact surface shaped to conform to a bone surface. Some embodiments also include a fluid capsule disposed in an interior region of the implant. Some embodiments have an insertion portion adapted to be inserted into a bone and a joint interface portion adapted to be disposed within a joint space, such as bone screws, sutures, or staples engaged with the IPN or semi-IPN and adapted to engage the bone to attach the IPN or semi-IPN to the bone and/or a stem extending from the bone contact surface and adapted to be inserted into the bone. The medical implant may also be incorporated as a bearing component of another device, such as a metal-based prosthesis.

The medical implant may also include a bonding agent adapted to attach the medical implant to a bone, such as a bone ingrowth surface formed on the bone contact surface. In some embodiments, the ionic polymer forms a concentration gradient from a first portion of the implant to a second portion of the implant. Some embodiments have a second hydrophobic thermoset or thermoplastic polymer adjacent to the first hydrophobic thermoset or thermoplastic polymer, the ionic polymer interpenetrating at least the first hydrophobic thermoset or thermoplastic polymer.

In some embodiments, the water-swellable IPN or semi-IPN has properties mimicking stiffness and lubricity properties of natural cartilage and may be adapted and configured to replace cartilage in a joint. The IPN or semi-IPN may have a shape selected from the group consisting of a cap, a cup, a plug, a mushroom, a stem, and a patch, and it may be adapted to fit a condyle, tibial plateau, meniscus, labrum, or glenoid.

The present disclosure also includes a method of repairing an orthopedic joint including the steps of replacing natural cartilage with a water-swellable IPN or semi-IPN having a hydrophobic thermoset or thermoplastic polymer and an ionic polymer and engaging the IPN or semi-IPN with a bone surface defining the joint. The method may also include the steps of bonding, suturing, stapling, and/or screwing the IPN or semi-IPN to the bone surface. The method may also include incorporating the material as a bearing component of another device, such as a metal-based prosthesis. The method may also include the step of inserting a stem portion into the bone surface. The orthopedic joint may be selected from a group consisting of a shoulder joint, a finger joint, a hand joint, an ankle joint, a foot joint, a toe joint, a knee medial compartment joint, a patellofemoral joint, a total knee joint, a knee meniscus, a femoral joint, an acetabular joint, a labral joint, an elbow, an intervertebral facet, and a vertebral joint.

The present disclosure also includes a marine hull coating including a water-swellable IPN or semi-IPN including a hydrophobic thermoset or thermoplastic polymer and an ionic polymer, the coating having a hull contact surface adapted to attach to a marine hull. The coating may also include an ultraviolet light protection agent and/or an anti-oxidation agent.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Figures 1A-D illustrate a method of forming an IPN or semi-IPN according to one aspect of this invention.
Figure 2 illustrates a composition gradient formed in an article along a thickness direction
Figure 3 illustrates a composition gradient formed in an article along a radial direction.
Figure 4A illustrates a method of fabricating a thermoplastic gradient IPN according to the present invention.
Figure 4B illustrates variation of gradient properties within an IPN according to the invention.
Figure 4C illustrates the variation of an ionic polymer across a gradient IPN.
Figure 5 illustrates a laminate structure or an IPN or semi-IPN.
Figures 6A-B illustrate shaping of a gradient IPN article.
Figures 7A-D illustrate shape heating of an IPN.
Figures 8A-Dillustrate bonding of a gradient IPN article to a surface.
Figures 9A-D illustrate how an osteochondral graft implant formed from an IPN or semi-IPN of this invention can be used to replace or augment cartilage within a joint.
Figures 10A-B illustrate an osteochondral graft having an opening to accommodate a ligament.
Figures 11A-E show osteochondral grafts formed from an IPN or semi-IPN of this invention that may be used singly or in any combination needed to replace or augment cartilage within a knee joint.
Figures 12A-B show osteochondral grafts formed from the IPN's or semi-IPN's of this invention and shaped for use in a finger joint.
Figures 13A-B show a labrum prosthesis formed from an IPN or semi-IPN of this invention for use in replacing or resurfacing the labrum of the shoulder or hip.
Figure 14 shows the use of an IPN or semi-IPN of this invention as a bursa osteochondral graft, labrum osteochondral graft, glenoid osteochondral graft and humeral head osteochondral graft.
Figure 15 shows the use of an IPN or semi-IPN of this invention as prostheses for resurfacing intervertebral facets.
Figure 16A shows a prosthetic cartilage plug formed from a gradient IPN composition of this invention.
Figures 16B-D show embodiments in which porous surfaces are formed on the cartilage plug. Figure 16D is a bottom elevational view of the embodiment of Figure 16C.
Figure 17 shows an embodiment of a prosthetic cartilage plug in which the stem is provided with helical ridges to form a screw for fixation of the plug to bone.
Figures 18A-B are side and bottom elevational views of an embodiment of a prosthetic cartilage plug having three stems for press fit insertion into holes in the bone for fixation.
Figure 19 shows an embodiment of a prosthetic cartilage plug in which the exposed head portion is substantially the same diameter as the stem.
Figure 20 shows an embodiment of a prosthetic cartilage plug in which the exposed head portion is narrower than the stem, and the stem widens toward the base.
Figure 21 shows an embodiment of a prosthetic cartilage plug in which the stem has circumferential ridges to aid fixation.
Figure 22 shows an embodiment similar to that of Figure 19 that adds a rough porous surface to the stem.
Figure 23 shows an embodiment of an osteochondral graft formed to physically grip the bone without additional fixation, such as screws or stems.
Figure 24 shows an embodiment of an osteochondral graft having screw holes for screw fixation.
Figure 25 shows an embodiment of an osteochondral graft having a screw hole and a screw head depression for screw fixation.
Figure 26 shows an embodiment of an osteochondral graft having a stem for insertion into a hole in the bone.
Figures 27A-B show embodiments of the composition of this invention used to make two-sided lubricious implants.
Figures 28 and 29 show orthopedic implants that are attached to surfaces of two bones or other anatomic elements that move with respect to each other, such as in a joint.
Figures 30A-B illustrate the integration of osteochondral grafts and other implants of this invention into bone over time.
Figures 31A-C illustrate three possible configurations of osteochondral implants to repair cartilaginous joint surface according to this invention.
Figure 32 shows the use of a lubricious IPN or semi-IPN composition of this invention to resurface the hull of a marine vessel.
Figure 33 shows the use of a lubricious thermoplastic or thermoset IPN to modify interfacing surfaces of machine parts that move with respect to each other.
Figure 34 shows the use of a lubricious thermoplastic or thermoset IPN to reduce fluid drag on the inner surface of a pipe.
Figure 35 is a photograph of a hydrated PEU/PAA semi-IPN gradient material being held by a forceps.
Figure 36 shows contact angle analysis in association with Example 32.
Figures 37A-B show the PEU/PAA semi-IPN material subject to Transmission Electron Microscopy analysis as associated with Example 33.
Figure 38 shows the PEU/PAA semi-IPN material subject to Transmission Electron Microscopy analysis with a schematic diagram associated with Example 34.
Figure 39 shows the tensile stress-strain behavior of the PEU/PAA semi-IPN material associated with Example 35.
Figure 40 shows the thermagram of the PEU/PAA semi-IPN material analyzed by DSC associated with Example 36.
Figure 41 shows the results of thermal analysis of the PEU/PAA semi-IPN material analyzed by DSC associated with Example 36.
Figure 42 shows the coefficient of friction of the PEU/PAA semi-IPN material on PEU/PAA under static load associated with Example 37.

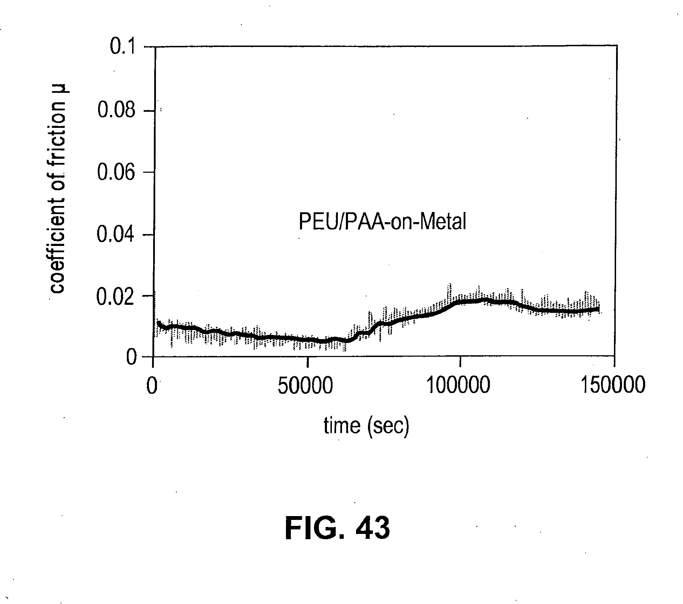

Figure 43 shows the coefficient of friction of the PEU/PAA semi-IPN material on metal under static load associated with Example 38.
Figures 44A-C show the results of wear testing of the PEU/PAA semi-IPN material associated with Example 39 compared to UHMWPE sample from a metal-on-UHMWPE wear test.
Figures 45A-C show the results of wear testing of the PEU/PAA semi-IPN material associated with Example 39.
Figure 46 shows quantification of the results of wear testing of the PEU/PAA semi-IPN material associated with Example 39.
Figure 47 shows the swelling behavior of polyether urethane and PEU/PAA semi-IPN in various aqueous and organic solvents associated with Example 40.
Figures 48A-B show the results of the swelling of polyether urethane and PEU/PAA semi-IPN in water and acetic acid associated with Example 41.
Figure 49 shows polyacrylic acid content in the PEU/PAA semi-IPN as a function of the amount of acrylic acid in the swelling solution associated with Example 42.

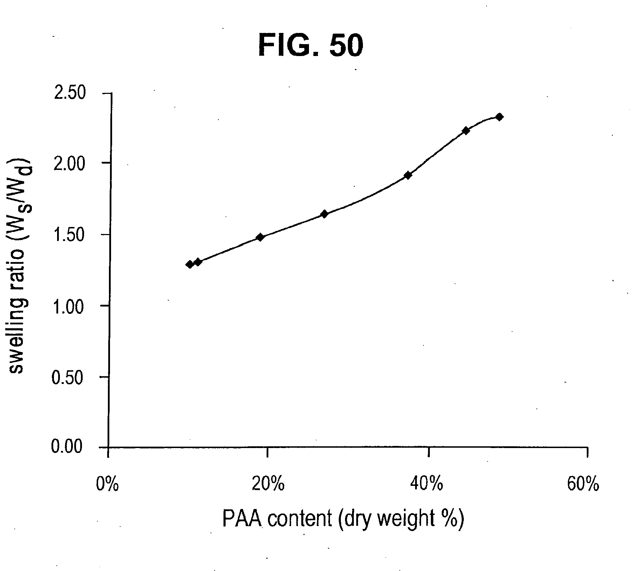

Figure 50 shows the swelling of PEU/PAA semi-IPN as a function of the amount of polyacrylic acid in the semi-IPN associated with Example 43.
Figures 51A-B show the results of Dynamic Compression testing of the PEU/PAA semi-IPN material as associated with Example 44.
Figure 52 shows the results of the application of a multistep stress relaxation compressive stress test to the PEU/PAA semi-IPN material followed by relaxation as associated with Example 44.
Figure 53 shows the results of the application of application of compressive stress to the PEU/PAA semi-IPN material associated with Example 44.
Figure 54 shows a partial list of materials that have been made in accordance with the present disclosure.

### DETAILED DESCRIPTION OF THE INVENTION

The present disclosure includes a process for modifying common commercially available hydrophobic thermoset or thermoplastic polymers to confer upon them qualities such as lubricity, permeability, conductivity and wear-resistance. Such hydrophobic polymers ordinarily do not soak up water and are generally useful for their mechanical strength, impermeability and insulating ability. An exemplary list of hydrophobic polymers modifiable by the process of this disclosure includes the following: Acrylonitrile butadiene styrene (ABS), Polymethylmethacrylate (PMMA), Acrylic, Celluloid, Cellulose acetate, Ethylene-Vinyl Acetate (EVA), Ethylene vinyl alcohol (EVAL), Kydex, a trademarked acrylic/PVC alloy, Liquid Crystal Polymer (LCP), Polyacetal (POM or Acetal), Polyacrylates (Acrylic), Polyacrylonitrile (PAN or Acrylonitrile), Polyamide (PA or Nylon), Polyamide-imide (PAI), Polyaryletherketone (PAEK or Ketone), Polyhydroxyalkanoates (PHAs), Polyketone (PK), Polyester, Polyetheretherketone (PEEK), Polyetherimide (PEI), Polyethersulfone (PES)- see Polysulfone, Polyethylenechlorinates (PEC), Polyimide (PI), Polymethylpentene (PMP), Polyphenylene oxide (PPO), Polyphenylene sulfide (PPS), Polyphthalamide (PPA), Polystyrene (PS), Polysulfone (PSU), Polyvinyl acetate (PVA), Polyvinyl chloride (PVC), Polyvinylidene chloride (PVDC), Spectralon, Styrene-acrylonitrile (SAN), Polydimethylsiloxane (PDMS), and Polyurethanes (PU). A wide variety of polyurethanes can be used with varying hard segment, soft segment, and chain extender compositions, as will be described herein.

One aspect of the disclosure takes advantage of a characteristic of some modifiable thermoset or thermoplastic hydrophobic polymers: The presence of ordered and disordered (amorphous) domains within the polymer. For example, some hydrophobic thermoset or thermoplastic polymers such as polyurethanes are phase-separated, containing first domains of hard segments and second domains of soft segments, with the two domains exhibiting different solubility properties with respect to interpenetration of monomers. In polyurethanes, the hard segments are disposed primarily within the ordered domains and the soft segments are disposed primarily within the disordered (amorphous) domains. (The starting polymer may contain more than two domains, of course, without departing from the scope of the invention.) This difference in properties between the two domains of the phase-separated polymer enables the process of this disclosure to impart new properties to the polymer that can extend throughout the bulk of the material or throughout only a portion of the material, e.g., in a particular region or in a gradient. For example, a non-lubricious polymer can be made lubricious; an otherwise non-conductive polymer can be made conductive; and an otherwise non-permeable polymer can be made permeable. Moreover, the process can be performed repeatedly to introduce more than one new property to the starting polymer.

In some embodiments, phase separation in the polymer allows for differential swelling of one or more separated phases within the polymer with, e.g., a solvent and/or monomer, which is then used to impart new properties. According to the invention, for example, lubriciousness can be introduced to an otherwise non-lubricious material by adding and polymerizing ionic monomers. In one embodiment, a polymer material with high mechanical strength and a lubricious surface can be made from an otherwise non-lubricious, hydrophobic polymer and a hydrophilic polymer derived from ionizable, vinyl monomers. By converting otherwise hydrophobic materials into biphasic materials with both solid and liquid (water) phases, the present invention addresses a need in the art for lubricious, high strength materials for use in medical, commercial, and industrial applications.

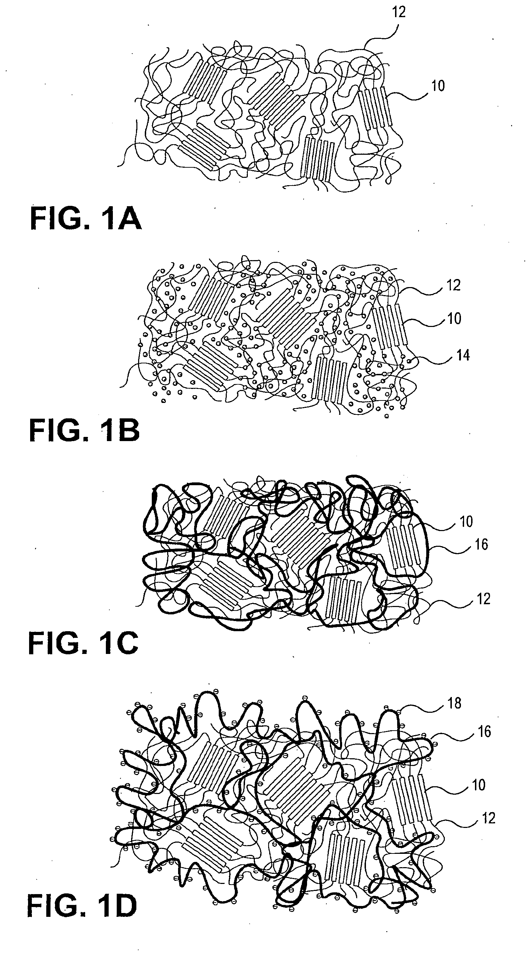

Figures 1A-D illustrate the process with respect to a thermoplastic polyurethane-based polymer containing a network of hard segments 10 (shown as open rectangles) and soft segments 12 (shown as lines). In Figure 1B, the soft segments 12 are swollen with vinyl-based monomer 14 (shown as circles) and optional solvent, along with an initiator and cross-linker (not shown), while mostly not affecting the hard segment material. This swelling process is not dissolution of the polymer; the hard segments act as physical crosslinks to hold the material together as the soft segments are imbibed with the monomer(s) and optional solvent(s). After polymerization and cross-linking of the monomers, a second network 16 (shown as dark lines in Figs. 1C and 1D) is formed in the presence of the first network to create an IPN in which the second polymer (i.e., the polymerized monomer) is primarily sequestered within the soft, amorphous domain of the first polymer. Despite some degree of molecular rearrangement and further phase separation, the hard segments largely remain ordered and crystalline, providing structure and strength to the material.

The new properties provided by this IPN depend on the properties of the polymerized monomers that were introduced and on any optional post-polymerization processing. Examples of such new properties include lubriciousness, conductivity, hardness, absorbency, permeability, photoreactivity and thermal reactivity. For example, as shown in Figure 1D, after optional swelling in a buffered aqueous solution, the second network of the IPN of Figure 1C becomes ionized 18, and the IPN is water-swollen and lubricious. Thus, hydrophilicity (i.e., water absorbency) can be introduced into an otherwise hydrophobic material. A hydrophobic polymer material such as polyurethane or ABS can be infiltrated with various ionic polymers such as polyacrylic acid and/or poly(sulfopropyl methacrylate) such that it absorbs water.

In addition to absorbency, various levels of permeability (water, ion, and/or solute transport) can be introduced into an otherwise non-permeable material. For example, a hydrophobic polymer material such as polyurethane or ABS can be infiltrated with an ionic polymer such as polyacrylic acid and/or poly(sulfopropyl methacrylate) so that it absorbs water, as described above. This hydration of the bulk of the material allows for the transport of solutes and ions. The transport of solutes and ions and permeability to water is made possible by phase continuity of the hydrated phase of the IPN. This is useful in various applications, including drug delivery, separation processes, proton exchange membranes, and catalytic processes. The permeability can also be utilized to capture, filter, or chelate solutes as a liquid flows over or through the material. Furthermore, because of this permeability, the materials of the present invention can be bestowed with increased resistance to creep and fatigue relative to their component hydrophobic polymers due to their ability to re-absorb fluid after sustained or repetitive loading.

Conductivity can be introduced into another wise non-conductive material. For example, an insulating polymer material such as polyurethane can be infiltrated with a conductive polymer (a polyelectrolyte) so that at least part of the hybrid material is conductive to electric current.

The invention also includes the alteration of chemical groups of the second polymer and the use of tethering points in the second polymer for another polymer, molecule or biomolecule. Also, any of the domains can be doped with any number of materials, such as antioxidants, ions, ionomers, contrast agents, particles, metals, pigments, dyes, biomolecules, polymers, proteins and/or therapeutic agents.

The first polymer can be additionally crosslinked or copolymerized with the second polymer if, for example, acryloxy, methacryloxy- acrylamido-, allyl ether, or vinyl functional groups are incorporated into one end or both ends of the polyurethane prepolymer and then cured by UV or temperature in the presence of an initiator. For instance, a polyurethane dimethacrylate or polyurethane bisacrylamide can be used in the first network by curing in the presence of a solvent (such as dimethylacetamide) and then evaporating the solvent. The addition of chemical crosslinks (rather than just physical crosslinks) to the IPN adds a level of mechanical stability against creep or fatigue caused by continuous, dynamic loading.

In addition, a multi-arm (multifunctional) polyol or isocyanate can be used to create crosslinks in the polyurethane. In this case, a fully interpenetrating polymer network is created (rather than a semi-interpenetrating polymer network). The result is a composite material with the high strength and toughness of polyurethane and the lubricious surface and biphasic bulk behavior of the poly(acrylic acid). Alternatively, other crosslinking methods can be used, including but not limited to gamma or electron-beam irradiation. These features are especially important for bearing applications such as artificial joint surfaces, or as more biocompatible, thrombo-resistant, long-term implants in other areas of the body such as the vascular system or the skin. Being swollen with water also allows imbibement with solutes such as therapeutic agents or drugs for localized delivery to target areas of the body.

In another embodiment of the present invention, the first polymer can be linked to the second polymer. For example, polyurethane can be linked through a vinyl-end group. Depending on the reactivity ratio between the end group and the monomer being polymerized, different chain configurations can be yielded. For instance, if the reactivity of the monomer with itself is much greater than the end group with the monomer, then the second polymer will be almost completely formed before the addition of the first polymer to the chain. On the other hand, if the reactivity of the monomer and the end group are similar, then a random grafting-type copolymerization will occur. The monomers and end groups can be chosen based on their reactivity ratios by using a table of relative reactivity ratios published in, for example, The Polymer Handbook. The result of these will be a hybrid copolymer/interpenetrating polymer network.

Any number or combinations of ethylenically unsaturated monomers or macromonomers (i.e., with reactive double bonds/vinyl groups) can be used alone or in combination with various solvents and selectively introduced into one or more of the phases of the polymer as long as at least 2% of such monomers is ionizable, i.e., contains carboxylic acid and/or sulfonic acid functional groups. Other monomers include but are not limited to dimethylacrylamide, acrylamide, NIPAAm, methyl acrylate, methyl methacrylate, hydroxyethyl acrylate/methacrylate, and any vinyl-based monomer containing sulfonic acid groups (e.g. acrylamido methyl propane sulfonic acid, vinyl sulfonic acid, 3-sulfopropyl acrylate (or methacrylate), 2-methyl-2-propene-1-sulfonic acid sodium salt 98%, or any monomers in which sulfonic acid is conjugated (allyl ethers, acrylate/methacrylates, vinyl groups, or acrylamides). The monomer can also include any monomers containing carboxylic acid groups conjugated to allyl ethers, acrylate/methacrylates, vinyl groups, or acrylamides. In addition, the monomers can be used in combination, such as both carboxyl acid and sulfonic acid containing monomers, to create a carboxylate/sulfonate copolymer. The pendant functional groups on polymers resulting from these monomers and monomer combinations can be subject to subsequent chemical reactions to yield other functionalities to the final polymer.

In one embodiment, a preformed, thermoplastic polymer may be immersed in acrylic acid (or in a solution of acrylic acid (1% - 100%) or other vinyl monomer solution) along with about 0.1% v/v crosslinker (e.g., triethylene glycol dimethacrylate or N,N methylene bisacrylamide) with respect to the monomer and about 0.1% v/v photoinitiator (e.g. 2-hydroxy-2-methyl propiophenone) with respect to the monomer. The acrylic acid solution can be based on water, salt buffer, or organic solvents such as dimethylacetamide, acetone, ethanol, methanol, isopropyl alcohol, toluene, dichloromethane, propanol, dimethylsulfoxide, dimethyl formamide, or tetrahydrofuran. The polymer may be swollen by the monomer due to solvation of the soft segments in the polymer. The monomer content in the swollen polymer can range from as little as about 1% to up to about 90%.

The monomer-swollen polymer may then be removed, placed in a mold made of glass, quartz, or a transparent polymer, then exposed to UV light (or elevated temperature) to initiate polymerization and crosslinking of the monomers. Alternatively, instead of using a mold, the monomer-swollen polymer can be polymerized while fully or partially exposed to air or an inert atmosphere (e.g., nitrogen or argon), or alternatively in the presence of another liquid such as an oil (e.g., paraffin, mineral, or silicone oil). For medical applications, it is possible that polymerization step can be performed in vivo without a mold.

Depending on the initiator used, exposure to UV light, IR, or visible light, a chemical, electrical charge, or elevated temperature leads to polymerization and crosslinking of the ionizable monomers within the hydrophobic polymer. As an example, acidic monomers (e.g. acrylic acid) are polymerized to form an ionic polymer within a preformed thermoplastic, hydrophobic matrix, forming an interpenetrating polymer network ("IPN"). Solvents can be extracted out by heat and convection or by solvent extraction. Solvent extraction involves the use of a different solvent (such as water) to extract the solvent from polymer, while heat or convection relies upon evaporation of the solvent. Depending on the pKa of the ionic polymer (e.g., pKa of PAA = 4.7), an acidic pH would leave the ionic polymer more protonated while a more basic pH would leave it more ionized.

Swelling of the IPN in aqueous solution such as phosphate buffered saline (or other buffered salt solution) at neutral pH will lead to ionization of the poly(acrylic acid) and further swelling with water and salts. The resulting swollen IPN will have a lubricious surface conferred by the hydrophilic, charged poly(acrylic acid) and high toughness and mechanical strength conferred by the thermoplastic. In the case of a polyurethane-based IPN, the IPN will have a structure in which crystalline hard segments in the polyurethane act as physical crosslinks in the first network, while chemical crosslinks will be present in the second network.

The materials can also be crosslinked after synthesis using gamma radiation or electron beam radiation. In one example, polyurethane/polyacrylic acid can be synthesized and then crosslinked by gamma irradiation, for instance with doses of, for example, 5, 10, 15, 20, or 25 kGy. In this case, the polymerization of polyacrylic acid would be done in the absence of a crosslinker, and after formation of the polymer blend (physical IPN), the material would be exposed to gamma radiation. This would have the dual purpose of sterilizing and crosslinking the polyurethane. It is known in the art that crosslinking of poly(acrylic acid) hydrogels using gamma irradiation shows a dose-dependence to the crosslinking of the polymer. This process can also be applied to other combinations of first and second network polymers, e.g., polyurethane and polymethyl methacrylate, ABS and polyacrylic acid.

In addition to the starting thermoset and thermoplastic hydrophobic polymers identified above, modifications to and derivatives of such polymers may be used, such as sulfonated polyurethanes. In the case of the polyurethanes, the polyurethane polymer can be a commercially available material, a modification of a commercially available material, or be a new material. Any number of chemistries and stoichiometries can be used to create the polyurethane polymer. For the hard segment, isocyanates used are 1,5 naphthalene diisocyanate (NDI), isophorone isocyanate (IPDI), 3,3-bitoluene diisocyanate (TODI), methylene bis (p-cyclohexyl isocyanate) (H₁₂MDI), cyclohexyl diiscocyanate (CHDI), 2,6 tolylene diisocyanate or 2,4 toluene diisocyanate (TDI), hexamethyl diisocyanate, or methylene bis(p-phenyl isocyanate). For the soft segment, chemicals used include, for example polyethylene oxide (PEO), polypropylene oxide (PPO), poly(tetramethylene oxide) (PTMO), hydroxy terminated butadiene, hydroxybutyl terminated polydimethylsiloxane (PDMS), polyethylene adipate, polycaprolactone, polytetramethylene adipate, hydroxyl terminate polyisobutylene, polyhexamethylene carbonate glycol, poly (1,6 hexyl 1,2-ethyl carbonate, and hydrogenated polybutadiene. Any number of telechelic polymers can be used in the soft segment, if end-groups that are reactive with isocyanates are used. For instance, hydroxyl- or amine- terminated poly(vinyl pyrrolidone), dimethylacrylamide, carboxylate or sulfonated polymers, telechelic hydrocarbon chains (with hydroxyl and/or amine end groups), dimethylolpropionic acid (DMPA), or these in combination with each other or with other soft segments mentioned above (e.g., PDMS) can be used. Ionic soft segments (or chain extenders) such as dihydroxyethyl propionic acid (DMPA) (or its derivatives) can be used to make a water-dispersible polyurethane, so long as the ionic chain extender does not comprise more than 2% of the material.

Chain extenders include, for example, 1,4 butanediol, ethylene diamine, 4,4'methylene bis (2-chloroaniline) (MOCA), ethylene glycol, and hexane diol. Any other compatible chain extenders can be used alone or in combination. Crosslinking chain extenders can be used containing isocyanate-reactive endgroups (e.g. hydroxyl or amine) and a vinyl-based functional group (e.g. vinyl, methacrylate, acrylate, allyl ether, or acrylamide) may be used in place of some or all of the chain extender. Examples incude 1,4 dihydroxybutene and glycerol methacrylate. Alternatively, crosslinking can be achieved through the use of a polyol such as glycerol which contains greater than two hydroxyl groups for reaction with isocyanates.

In some embodiments, at least 2% of the hydrophilic monomers in the second network is ionizable and anionic (capable of being negatively charged). In one such embodiment, poly(acrylic acid) (PAA) hydrogel is used as the second polymer network, formed from an aqueous solution of acrylic acid monomers. Other ionizable monomers include ones that contain negatively charged carboxylic acid or sulfonic acid groups, such as methacrylic acid, 2-acrylamido-2-methylpropanesulfonic acid, sulfopropyl methacrylate (or acrylate), vinyl sulfonic acid, or vinyl-conjugated versions of hyaluronic acid, heparin sulfate, and chondroitin sulfate, as well as derivatives, or combinations thereof. The second network monomer may also be positively charged or cationic. These other monomers can also be in a range of 1% - 99% in either water or organic solvent, or be pure (100%). One embodiment of the monomer used to form the second network can be described by the following characteristics: (1) it is capable of swelling the polyurethane, (2) capable of polymerizing, and (3) is ionizable.

Other embodiments use a co-monomer in addition to the ionic polymer that may be non-ionic, such as acrylamide, methacrylamide, *N*-hydroxyethyl acrylamide, *N*-isopropylacrylamide, methylmethacrylate, *N*-vinyl pyrrolidone, 2-hydroxyethyl methacrylate, 2-hydroxyethyl acrylate or derivatives thereof. These can be copolymerized with less hydrophilic species such as methylmethacrylate or other more hydrophobic monomers or macromonomers. These can also be polymerized alone or copolymerized with the aforementioned hydrophilic and/or ionizable monomers.

Crosslinked linear polymer chains (i.e., macromolecules) based on these monomers may also be used in the second network, as well as biomacromolecules (linear or crosslinked) such as proteins and polypeptides (e.g., collagen, hyaluronic acid, or chitosan). The choice of the second material will depend on the target application, for instance in orthopaedic applications, hyaluronic acid may be useful because it is a major component of joint cartilage. In addition, biological molecules may carry certain benefits such as intrinsic biocompatibility or therapeutic (e.g., wound healing and/or antimicrobial) properties that make them useful as material components.

Any type of compatible cross-linkers may be used to crosslink the second network in the presence of any of the aforementioned first networks such as, for example, ethylene glycol dimethacrylate, ethylene glycol diacrylate, diethylene glycol dimethacrylate (or diacrylate), triethylene glycol dimethacrylate (or diacrylate), tetraethylene glycol dimethacrylate (or diacrylate), polyethylene glycol dimethacrylate, or polyethylene glycol diacrylate, methylene bisacrylamide, *N,N'*-(1,2-dihydroxyethylene) bisacrylamide, derivatives, or combinations thereof. Any number of photoinitiators can also be used depending on their solubility with the precursor solutions/materials. These include, but are not limited to, 2-hydroxy-2-methyl-propiophenone and 2-hydroxy-1-[4-(2-hydroxyethoxy) phenyl]-2-methyl-1-propanone. In addition, other initiators such as benzoyl peroxide, 2-oxoglutaric acid, azobisisobutyronitrile, or potassium persulfate (or sodium persulfate) can be used. For instance, benzoyl peroxide is useful for temperature-initiated polymerizations, while azobisisobutyronitrile and sodium persulfate are useful as radical initiators.

In another embodiment, a solvent can be used as a "trojan horse" to deliver monomers that otherwise would not mix (or solubilize with) the polymer to one (or more) phases of the polymer. The solvent must be carefully chosen based on the specific qualities and phases of the polymer and monomers. For instance, acetic acid is capable of swelling but does not dissolve many polyurethanes. Therefore, acetic acid can be used to carry other monomers such an acrylamide solution, that otherwise would not enter polyurethane, into the bulk of the polyurethane. This allows the acrylamide to be selectively polymerized inside one phase of the polyurethane. The acetic acid can then be washed out leaving behind a polyurethane with one or more new properties. Other solvents that can be used include, but are not limited to, dichloromethane, methanol, propanol, butanol, (or any alkyl alcohol), acetone, dimethylacetamide, dimethylformamide, dimethylsulfoxide, tetrahydrofuran, diethylether, or combinations of these. Taking into account the solubilities in the phases of the polymer, solvents with varying degrees of swelling of one can be chosen. Solubilities of the solvents and components of the material to be swollen can be obtained from polymer textbooks such as The Polymer Handbook or can be measured experimentally.

The present invention can be used to form a bulk-interpenetrated coating on a polymeric material. This coating is inextricably entangled with the underlying polymer matrix, and is in contrast to conventional surface coatings in which a material is grafted or tethered to a surface. In one example of a bulk-interpenetrated coating, a thermoplastic polymer is coated on one or more sides or is immersed in an ionizable monomer such as acrylic acid in the presence of a photoinitiator and a crosslinking agent. The thermoplastic is then placed in a mold and then exposed to an initiator (e.g., UV light or heat) for a predetermined period of time. The mold can be fully or partially transparent and/or masked to facilitate regionally specific curing of the monomer. The modified material is then immersed in buffered saline solution to neutralize the ionic polymer and render the surface lubricious and hydrophilic. The modified plastic can then be further remolded by application of heat, solvent, and/or pressure and then shaped to the desired dimensions. The modified plastic can then be bonded to various surfaces such as metal, glass, plastic, or other materials by applying heat or solvent (such as acetone) to the unmodified plastic surface and bringing the surface in contact with the surface of interest.

Among the applications of the invention are the creation of hydrophilic, lubricious sidings or coatings to reduce drag and/or biofilm formation and/or barnacle formation in marine vessels, diving or swimming suits, other water crafts or water-borne objects, or pipes. In addition, the invention can be used as a method for making bearings and moving parts for applications such as engines, pistons, or other machines or machine parts. The invention can also be used in artificial joints systems or long-term implants in other areas of the body, such stents and catheters for the vascular or urinary system or implants, patches, or dressings for the skin.

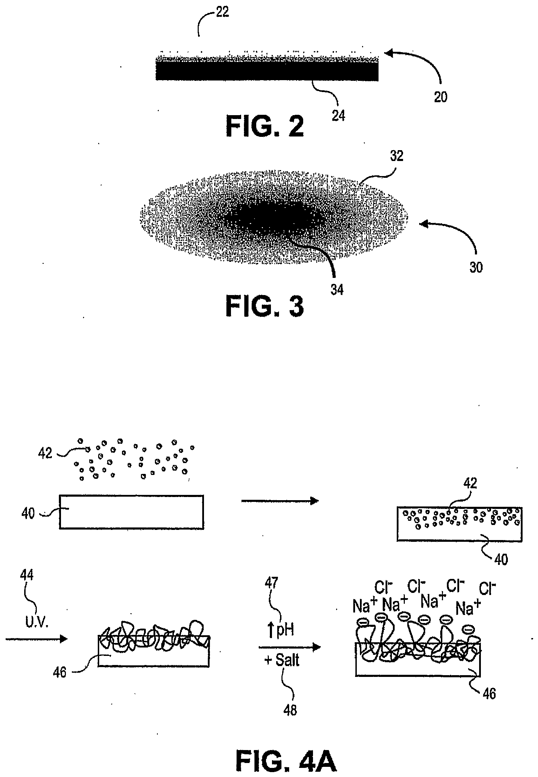

Figures 2 and 3 illustrate how the invention can be used to create a composition gradient within a starting homopolymer. In Figure 2, a gradient is formed in material 20 along a thickness direction, with the IPN formed on one side 22 and extending in a diminishing concentration to another side 24, e.g., substantially only homopolymer. In Figure 3, the IPN concentration gradient is radial within material 30, with the outer surface 32 being the highest concentration of IPN and the center or core 34 having the lowest concentration of IPN. A reverse gradient can also be made in the case of a cylinder or a sphere, with the IPN disposed in the core of the shape and the hydrophobic polymer being disposed in the outer aspect of the shape. This is useful in creating a conductive semi-IPN wire that is encapsulated within an insulating hydrophobic material via a gradient composition.

Figure 4A illustrates a method of fabricating a thermoplastic gradient IPN according to the present invention. One side of the thermoplastic material 40 is imbibed with a monomer solution 42 along with a photoinitiator (not shown) and a crosslinker (not shown), and then the monomer is polymerized and crosslinked (e.g., with UV light 44) within the thermoplastic to form a gradient IPN 46. Increasing the pH to neutral 47 and introducing salt 48 into the surrounding fluid leads to ionization of the 2nd polymer network. Alternatively, non-ionic monomers can be used as the basis in a part (to form a copolymer). The non-ionic polymer would not be ionized by the buffer solution, but would still create a hydrophilic surface. Either type of monomer system can be used in conjunction with either water or an organic solvent.

In one embodiment, a TP/PAA IPN can be created in a gradient if polyurethane ("PU") is swollen in AA on one side only or if the swelling time is limited such that diffusion of the monomers through the bulk of the TP is not complete. This is especially useful in the creation of osteochondral grafts for orthopaedic joint replacement materials. For instance, in the case of a cartilage replacement material, one side of the material is made lubricious and water swollen, while the other remains a solid (pure thermoplastic). In between is a transition between a TP/PAA IPN and TP, with decreasing PAA content from one surface to the other. Alternatively, bulk materials with a TP/PAA IPN outer aspect and PU-only "core" can be made if the diffusion of AA into the TP is precisely controlled by timing the infiltration of the monomers into the bulk. The differential swelling that results from this configuration can lead to remaining stresses (compressive on the swollen side, tensile on the non-swollen side) that can help enhance the mechanical and fatigue behavior of the material. In the case of a material with a thickness gradient, the base of thermoplastic-only material can be used for anchoring, adhering, or suturing the device to the anatomical region or interest. This base can be confined to a small area or be large (e.g., a skirt) and can extend outward as a single component or multiple components (e.g., straps). The internal stresses built up within the thermoplastic during processing or after swelling can be reduced by temperature-induced annealing. For instance, temperatures of 60 - 120 degrees Celsius can be used for various times (30 minutes to many hours) to anneal the polymer, and the heat can be applied in an oven, by a hot surface, by radiation, or by a heat gun. The thermoplastic can later be crosslinked using, for example, gamma or electron beam radiation.

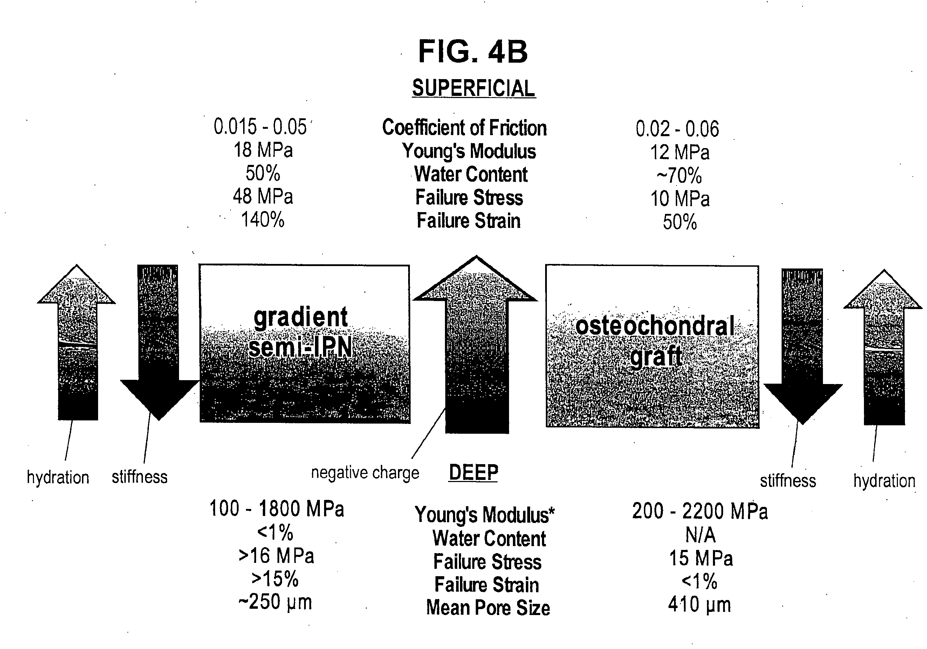

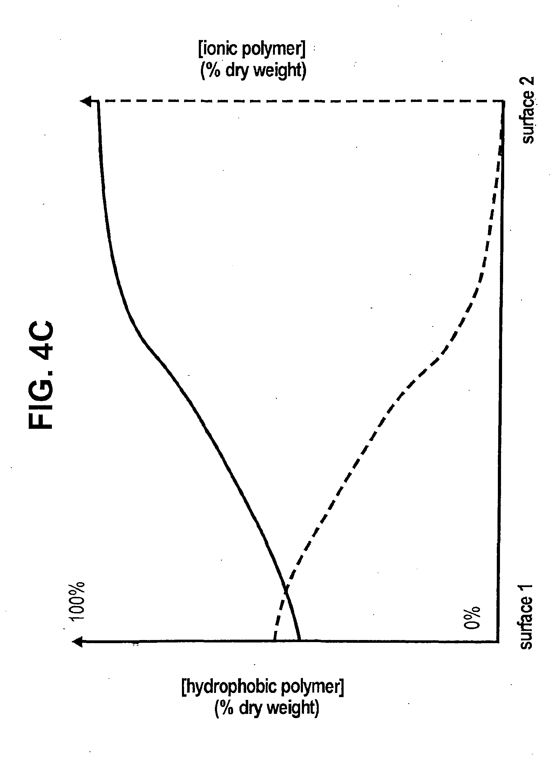

Figure 4B illustrates how the properties of gradient IPN's can vary to produce the desired composition. Figure 4C illustrates how the concentration gradient of the hydrophobic polymer and the ionic polymer can vary across the thickness (between the two surfaces) of a gradient IPN. The composition gradient yields a property gradient in which the IPN is hydrated and more compliant on one side, and less hydrated (or not hydrated) and stiff on the other.

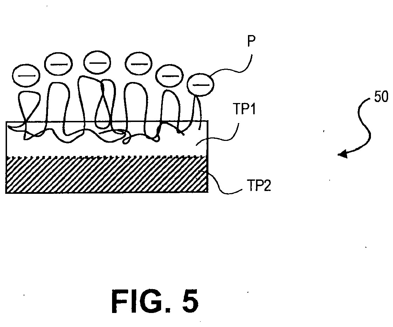

Articles made from the IPN's and semi-IPN's of this invention may also be formed in a laminate structure, as illustrated in Figure 5. In one example, the IPN structure 50 is comprised of a hydrophilic polymer (P) such as poly(acrylic acid) that is interpenetrating a first thermoplastic (TP1) such as polyether urethane, which is formed on top of a second thermoplastic (TP2) such as polycarbonate urethane. Both TP1 and TP2 can be themselves comprised of multiple layers of various hardnesses and properties. In addition, many more than two thermoplastic layers can be used, and one or more of the thermoplastics can be crosslinked. Finally, non-thermoplastic elements can be incorporated into this construct.

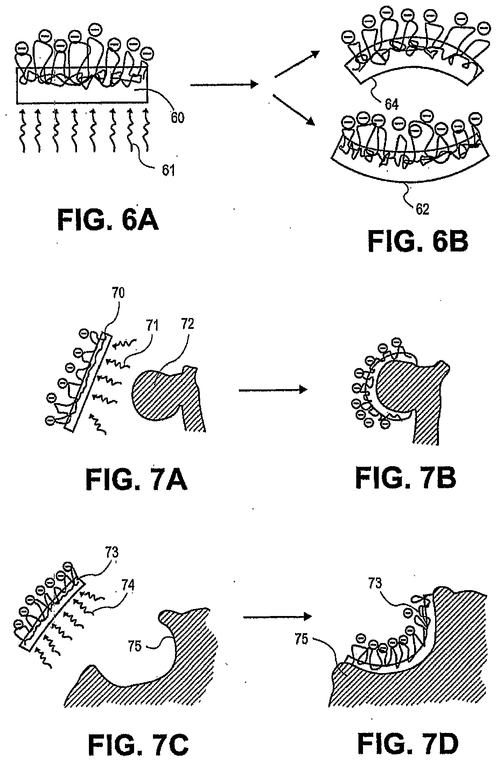

Articles formed from the gradient or homogeneous IPN's and semi-IPN's of this invention may be shaped as desired. Figure 6 illustrates shaping of a gradient IPN article. This process may also be used to shape a homogeneous IPN or semi-IPN.

As shown in Figures 6A, heat 61 can be used to re-anneal the physical crosslinks in the polymer (e.g., the hard segments in the polyurethane) in the thermoplastic side 60 of the gradient IPN to lead to different desired curvatures after bending (e.g., over a mold or template) and cooling. Figure 6B illustrates both convex 62 and concave 64 curvatures on the thermoplastic side of the gradient IPN. Other shapes may be formed, of course, as desired. The use of thermoplastic facilitates molding of a device to a desired shape by, for example, injection molding, reactive injection molding, compression molding, or alternatively, dip-casting. The molded device can then be subjected to subsequent network infiltration and polymerization steps to yield the new IPN material.

Shaping of IPN and semi-IPN articles according to this invention may be formed *in situ,* such as within a human body. For example, Figures 7A-B illustrate heating 71 of a thermoplastic gradient IPN 70 to enable it to wrap around the curvature of a femoral head 72. Figures 7C-D illustrate the application of heat 74 to a thermoplastic gradient IPN 73 to enable it to adapt to the curvature of a hip socket 75.

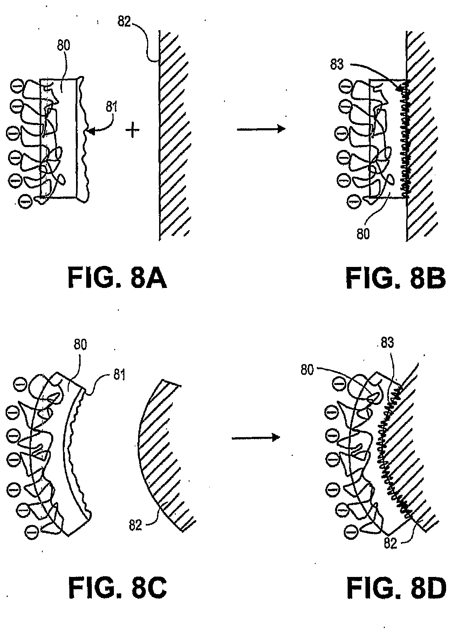

Shaped or unshaped IPN and semi-IPN articles made according to this invention may be attached to other surfaces. Figure 8A-D shows how a bonding agent 81 such as a solvent, cement, or glue can be used to attach the thermoplastic gradient IPN article 80 to a surface 82 at a bonded interface 83. Addition of the solvent, for example, causes the material to dissolve locally, and after contact with a surface and drying of the solvent, the thermoplastic adheres to the surface. This method can be used to create "paneling" of the present invention of various objects, including but not limited to marine vessel hull surfaces. A "coating" can be applied by by vacuum forming the material over the contours of the vessel or a part of the vessel. A similar approach can be used to attach a gradient IPN to bone surfaces in joints.

The composition of this invention, formed, e.g., by the method of this disclosure, may be used in a variety of settings. One particular use is as artificial cartilage in an osteochondral graft. The present invention provides a bone-sparing arthroplasty device based on an interpenetrating polymer network that mimics the molecular structure, and in turn, the elastic modulus, fracture strength, and lubricious surface of natural cartilage. Emulating at least some of these structural and functional aspects of natural cartilage, the semi-IPNs and IPNs of the present invention form the basis of a novel, bone-sparing, "biomimetic resurfacing" arthroplasty procedure. Designed to replace only cartilage, such a device is fabricated as a set of flexible, implantable devices featuring lubricious articular surfaces and osteointegrable bone-interfaces.

In principle, the device can be made for any joint surface in the body. For example, a device to cover the tibial plateau will require an analogous bone-preparation and polymer-sizing process. For a device to cover the femoral head in the hip joint, a cap shaped device fits snugly over the contours of the femoral head. For a device to line the acetabulum, a hemispherical cup-shaped device stretches over the lip and can be snapped into place in the socket to provide a mating surface with the femoral head. In this way, both sides of a patient's hip joint can be repaired, creating a cap-on-cap articulation. However, if only one of the surfaces is damaged, then only one side may be capped, creating a cap-on-cartilage articulation. In addition, the materials of the present invention can be used to cap or line the articulating surfaces of another joint replacement or resurfacing device (typically comprised of metal) to serve as an alternative bearing surface.

To create a cap-shaped device using the present invention for the shoulder joint (also a ball-and-socket joint), a process similar to that of the hip joint is used. For instance, a shallow cup can be created to line the inner aspect of the glenoid. Furthermore, devices for other joints in the hand, fingers, elbow, ankles, feet, and intervertebral facets can also be created using this "capping" concept. In one embodiment in the distal femur, the distal femur device volume follows the contours of the bone while sparing the anterior and posterior cruciate ligaments.

In one embodiment of prosthetic cartilage formed according to this invention, a polyether urethane device pre-formed with shore hardness of 75D is injection molded. This device is then solution casted in a Vitamin E-containing solution containing polyether urethane formulated to a dry shore hardness of 55D (e.g., 25% Elasthane™ 55D in dimethylacetamide). The casted layer may then be dried in a convection oven to remove the solvent. The device may then be immersed in a solution of acrylic acid, photoinitiator, and crosslinker for 24 hours, and then placed over a glass mold and exposed to UV light. The resulting device may then be soaked and washed in phosphate buffered saline. This process is used to create either convex or concave devices for arthroplasty applications. The injection-molded pre-form has on one of its sides a plurality of spaces (pores or features) that make capable of being anchored to bone with traditional orthopaedic bone cement.

In another embodiment of the device, a polycarbonate urethane pre-formed with surface features on one side is fabricated, followed by dip-casting of one of its sides in a solution of polyether urethane and then subjected to a process similar to the one above. In still another embodiment, a polyether urethane pre-form of shore hardness 55D (e.g., Elasthane™ 55D) is injection molded, followed by immersion in a monomer solution as above. After curing of the second polymer network, the device is dip-casted on one side with polycarbonate urethane of shore hardness 75D. In any of these embodiments, additional surface features can be added to the bone interface side of the device through a number of means, including but not limited to machining (lathe and end-mill), solution casting, solvent-welding, ultrasonic welding, or heat-welding.

Porous polycarbonate urethane IPN and semi-IPN structures may be made according to this invention. Particles (size range: 250-1500 µm) of polycarbonate urethane, including but not limited to Bionate® 55D, Bionate® 65D, and Bionate® 75D, may be sintered in a mold using heat (220-250°C), pressure (0.001-100 MPa), and/or solvent for 10-30 min. The structures will have a final pore size of 50-2000 µm, porosity of 15-70%, and a compressive strength exceeding 10 MPa. The final structures will have porosity to promote tissue ingrowth/integration for medical and veterinary applications. This construct can be used alone or with an overlying bearing surface made from any of the lubricious polymers described in this invention. This material could be used as a cartilage replacement plug in joints of the body where cartilage has been damaged, as described below.

The composition of this invention, made, e.g., according to the method of this disclosure, may be used as a fully or partially synthetic osteochondral graft. The osteochondral graft consists of a lubricious, cartilage-like synthetic bearing layer that may be anchored to porous bone or a synthetic, porous bone-like structure. The bearing layer has two regions: a lubricious surface layer and a stiff, bone anchoring layer. In one embodiment, the top, lubricious region of the bearing layer consists of an interpenetrating polymer network that is composed of two polymers. The first polymer may be a hydrophobic thermoplastic with high mechanical strength, including but not limited to polyether urethane, polycarbonate urethane, silicone polyether urethane, and silicone polycarbonate urethanes, or these materials with incorporated urea linkages, or these materials with incorporated urea linkages (e.g. polyurethane urea). The second polymer may be a hydrophilic polymer derived from ionizable, vinyl monomers, including but not limited to acrylic acid and/or sulfopropyl methacrylate. The bottom region of the bearing layer (bone anchoring layer) may be a stiff, non-resorbable thermoplastic that can be caused to flow with ultrasonic welding vibration, ultrasonic energy, laser energy, heat, RF energy and electrical energy. The bone anchoring layer is used to anchor the bearing layer to bone or a bone-like porous structure. If porous bone is used, it can be cancellous bone from a human or animal. If a synthetic bone-like material is used, it can consist of porous calcium-phosphate (and/or other materials, including but not limited to porous carbonated apatite, beta-tricalcium phosphate, or hydroxyapatite), or a porous resorbable or non-resorbable thermoplastic as described above, including but not limited to polycarbonate urethane, polyether urethane, PLA, PLLA, PLAGA, and/or PEEK. The bearing layer is anchored to the porous bone or bone-like structure via application of pressure combined with energy that cause the bone anchoring material to melt and flow into the pores or spaces of the bone or bone-like structure, after which the energy source is removed and the material resolidifies. The energy source can include but is not limited to vibration, ultrasonic energy, laser energy, heat, RF energy, and electrical energy.

The following figures illustrate various embodiments of the present invention as a device to partially or completely resurface damaged joints in the body of mammals (animals or human). These devices can be fixated to bone through any number of means, such as a press-fit, screws (metal or plastic, either resorbable or nonresorbable), sutures (resorbable or nonresorbable), glue, adhesives, light-curable adhesives (e.g. polyurethane or resin-based), or cement (such as polymethylmethacrylate or calcium phosphate, or dental cements).

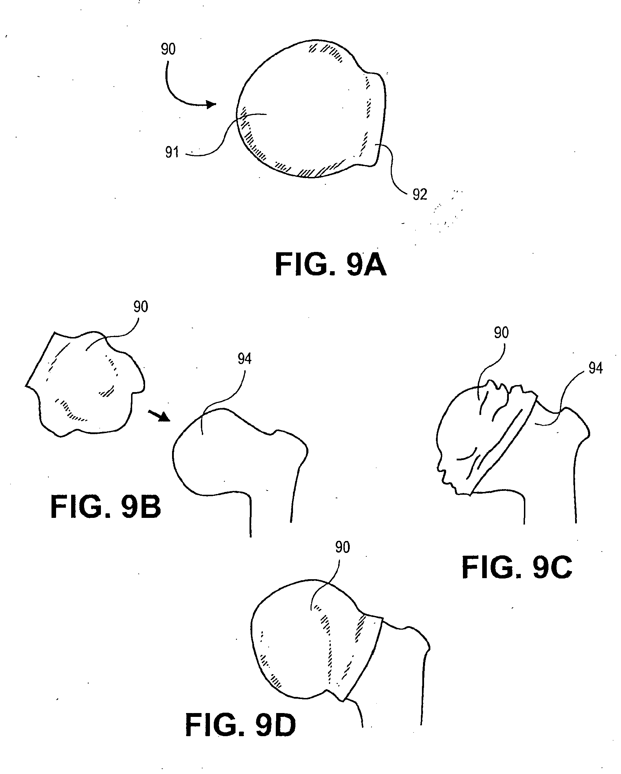

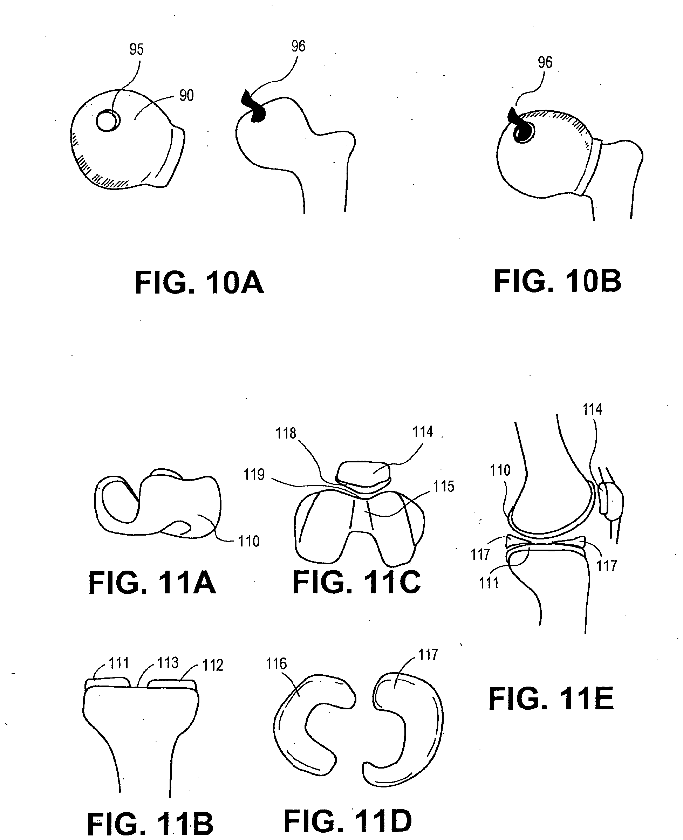

Figures 9A-D illustrate how an osteochondral graft implant formed from an IPN or semi-IPN of this invention can be used to replace or augment cartilage within a joint, such as a hip or shoulder joint. As shown in Figure 9A, the prosthetic cartilage 90 is formed as a sock having a cap portion 91 and an optional collar 92. The prosthesis 90 may be inverted, as shown in Figure 9B, and slipped over the head 94 of the humerus or femur. In an alternative embodiment shown in Figures 10A-B, the prosthesis 90 may include an opening 95 to accommodate a ligament 96 or other anatomical structure.

Implants and other articles may be made in a variety of complex shapes according to the invention. Figures 11A-E show osteochondral grafts formed from an IPN or semi-IPN of this invention that may be used singly or in any combination needed to replace or augment cartilage within a knee joint. Figure 11A shows a osteochondral graft 110 adapted to engage the femoral condyles (or alternatively, just one condyle). Figure 11B shows osteochondral grafts 111 and 112 adapted to engage one or both sides of the tibial plateau 113. Figure 11C shows an osteochondral graft 118 adapted to engage the patella 114 and to articulate with an osteochondral graft 119 adapted to engage the patellofemoral groove 115. Figure 11D show osteochondral grafts 116 and 117 adapted to engage the lateral and medial menisci. Figure 11E shows how some of these prostheses may be assembled in place within the knee joint.

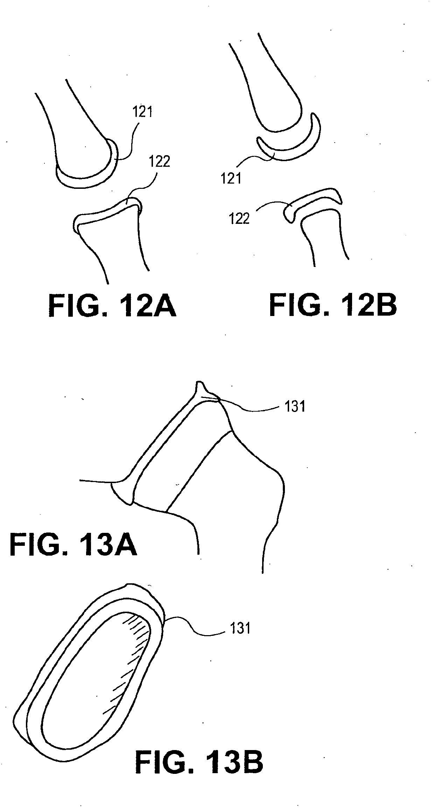

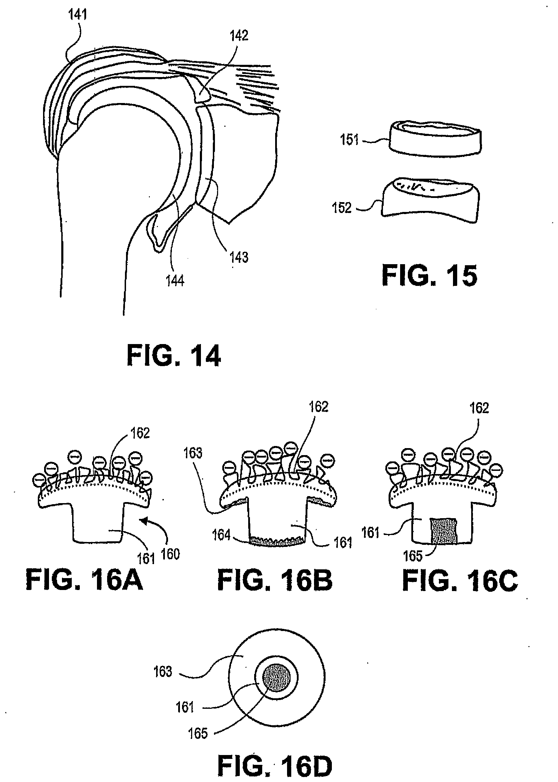

Osteochondral grafts may also be used in other joints, such as in the finger, hand, ankle, elbow, feet or vertebra. For example, Figures 12A-B show osteochondral grafts 121 and 122 formed from the IPN's or semi-IPN's of this invention and shaped for use in a finger joint. Figures 13A-B show a labrum prosthesis 131 formed from an IPN or semi-IPN of this invention for use in replacing or resurfacing the labrum of the shoulder or hip. Figure 14 shows the use of an IPN or semi-IPN of this invention as a bursa osteochondral graft 141, labrum osteochondral graft 142, glenoid osteochondral graft 143 and humeral head osteochondral graft 144. Figure 15 shows the use of an IPN or semi-IPN of this invention as prostheses 151 and 152 for resurfacing intervertebral facets.

The IPN's and semi-IPN's compositions of this invention may be formed as prosthetic cartilage plugs for partial resurfacing of joint surfaces. Figure 16A shows a prosthetic cartilage plug 160 formed from a gradient IPN composition of this invention. Plug 160 has a stem portion 161 formed on a thermoplastic side of the article and adapted to be inserted into a hole or opening in a bone. The head 162 of the plug is formed to be a lubricious IPN or semi-IPN, as described above. Figure 16B shows a variation in which porous surfaces are formed on the underside 163 of head 162 and on the base 164 of stem 161. In the embodiment of Figures 16C-D, the porous suface is formed only in the center portion 165 of base 164. In all embodiments, stem 161 may be press fit into a hole or opening in the bone, leaving the lubricious IPN surface to be exposed to act as prostethic cartilage.

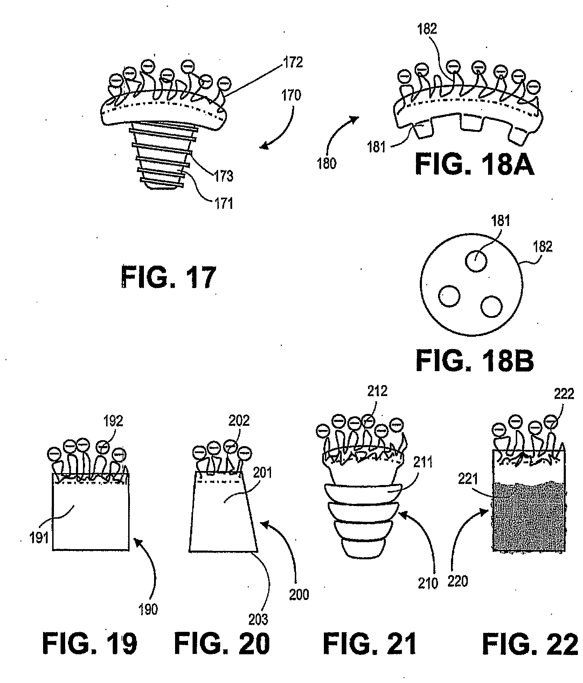

Figure 17 shows an embodiment of a prosthetic cartilage plug 170 in which the stem 171 is provided with helical ridges 173 to form a screw for fixation of the plug to bone. The top surface of the head 172 is a lubricious IPN or semi-IPN, as above.

Figures 18A-B shows an embodiment of a prosthetic cartilage plug 180 having three stems 181 for press fit insertion into holes in the bone for fixation. The top surface of plug head 182 is a lubricious IPN or semi-IPN, as above.

Figure 19 shows an embodiment of a prosthetic cartilage plug 190 in which the exposed head portion 192 is substantially the same diameter as the stem 191. Stem 191 may be press fit into a hole in the bone for fixation. The top surface of plug head 192 is a lubricious IPN or semi-IPN, as above.

Figure 20 shows an embodiment of a prosthetic cartilage plug 200 in which the exposed head portion 202 is narrower than stem 201, and stem 201 widens toward base 203. Stem 201 may be press fit into a hole in the bone for fixation. The top surface of plug head 202 is a lubricious IPN or semi-IPN, as above.

Figure 21 shows an embodiment of a prosthetic cartilage plug 210 in which the stem 211 has circumferential ridges to aid fixation. Stem 211 may be press fit into a hole in the bone for fixation. The top surface of plug head 212 is a lubricious IPN or semi-IPN, as above.

Figure 22 shows an embodiment similar to that of Figure 19 that adds a rough porous surface to stem 221. The top surface of plug head 222 is a lubricious IPN or semi-IPN, as above.

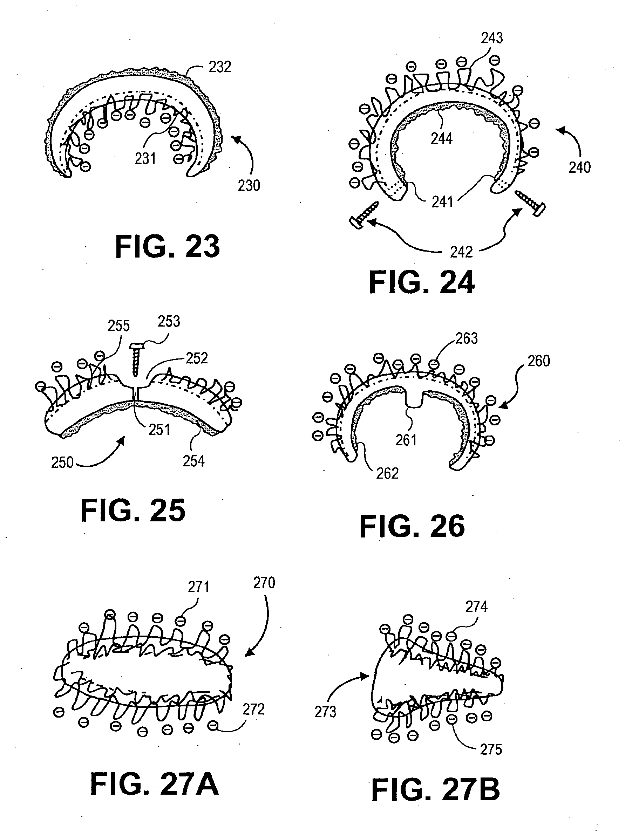

Figure 23 shows an embodiment of an osteochondral graft 230 formed to physically grip the bone without additional fixation, such as screws or stems. In this embodiment, the lubricious IPN or semi-IPN portion of the prosthesis is on a concave surface 231 of the device. The opposite convex surface 232 of the device is shaped to match the shape of the bone to which prosthesis 230 will be attached. Surface 232 is porous to facilitate bony ingrowth. The porous material in this case can be fabricated from a porogen method as described in the present invention, with the porogen being sodium chloride, tricalcium phosphate, hydroxyapatite, sugar, and derivatives or combinations thereof. Alternatively, the porosity can be derived from sintering polymer beads (e.g. polyether urethane or polycarbonate urethane) together using heat or solvent.

Screw holes may be provided to the osteochondral graft for fixation to the bone. In Figure 24, prosthesis 240 is provided with two holes 241 for screws 242. The bone-contacting concave side 244 of prosthesis 240 is porous (as above) to promote bony ingrowth and has a shape adapted for physically gripping the bone. The outer convex surface 243 of the prosthesis is a lubricious IPN or semi-IPN, as above.

In Figure 25, the osteochondral graft 250 is provided with a screw hole 251 as well as a depression 252 for accommodating the head of a screw 253. The bone-contacting concave side 254 of prosthesis 250 is porous (as above) to promote bony ingrowth and has a shape adapted for physically gripping the bone. The outer convex surface 255 of the prosthesis is a lubricious IPN or semi-IPN, as above.

Figure 26 shows an embodiment of an osteochondral graft 260 having a stem 261 for insertion into a hole in the bone. The bone-contacting concave side 262 of prosthesis 260 is porous (as above) to promote bony ingrowth and has a shape adapted for physically gripping the bone. The outer convex surface 263 of the prosthesis is a lubricious IPN or semi-IPN, as above.

Figures 27A-B show embodiments of the composition of this invention used to make two-side lubricious implants. In Figure 27A, implant 270 is sized and configured to replace an intervertebral disc. Implant 270 has lubricious IPN or semi-IPN surfaces 271 and 272 (formed, e.g., as described above) on its upper and lower sides. Figure 27B shows a knee spacer 273 having a wedge-shaped cross-section. As with disc prosthesis 270, spacer 273 also has lubricious IPN or semi-IPN surfaces 274 and 275 on its upper and lower sides.

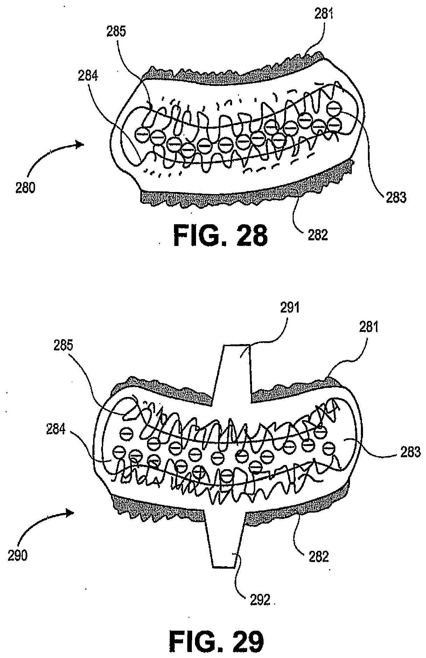

Many of the osteochondral grafts and other implants described above are affixed to a single bone surface. Figures 28 and 29 show orthopedic implants that are attached to surfaces of two bones or other anatomic elements that move with respect to each other, such as in a joint. In Figure 28, implant 280 has upper and lower bone contacting regions 281 and 282 formed to be porous (as described above) to promote bony ingrowth. The interior of implant 280 is a fluid-filled capsule 283. Inwardly facing bearing surfaces 284 and 285 are lubricious IPN or semi-IPN surfaces (as above). Implant 280 can be used, e.g., as an interpositional spacer and as a replacement for the synovial capsule and cartilage of a joint. The implant 290 of Figure 29 is similar to that of Figure 28, but adds upper and lower stems 291 and 292 for insertion and fixation in corresponding holes in the bones defining the joint.

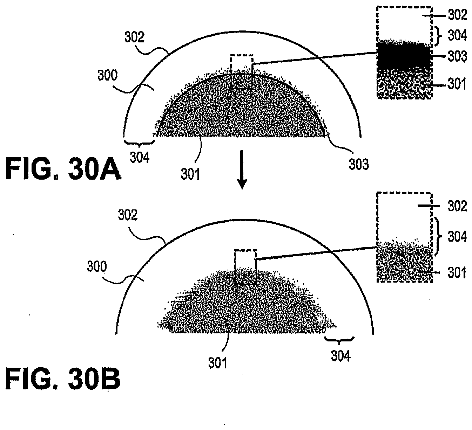

Figures 30A-B illustrate the integration of osteochondral grafts and other implants of this invention into bone over time. In Figure 30A, an osteochondral graft implant 300 formed as described above is placed over bone 301. Implant 300 has a lubricious IPN or semi-IPN surface 302 and a bone interface surface 303 formed from a thermoset or thermoplastic hydrophobic polymer alone, which is optionally porous as described above. Between surface 302 and surface 303 is a gradient or transition zone 304 between the IPN or semi-IPN and the hydrophobic polymer. Over time, bone tissue will grow from bone 301 into and through the bone contacting surface 303, as illustrated in Figure 30B.

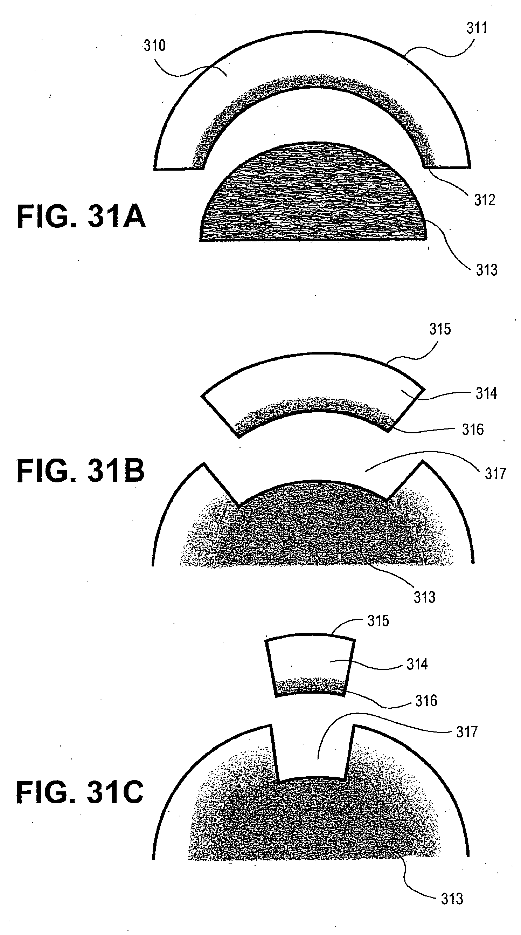

Figures 31A-C illustrate three possible configurations of osteochondral implants to repair cartilaginous joint surface according to this invention. In Figure 31A, implant 310 is formed as a cap having a lubricious IPN or semi-IPN surface 311 transitioning to a bone-contacting surface 312 formed from a thermoset or thermoplastic hydrophobic polymer, as described above. When implanted, implant 310 covers the outer surface of bone 313.

Figures 31B and 31C show configurations in which implant 314 is formed as a patch or plug (respectively) having a lubricious IPN or semi-IPN surface 315 transitioning to a bone-contacting surface 316 formed from a thermoset or thermoplastic hydrophobic polymer, as described above. When implanted, implant 314 fits within a prepared opening 317 of bone 313.

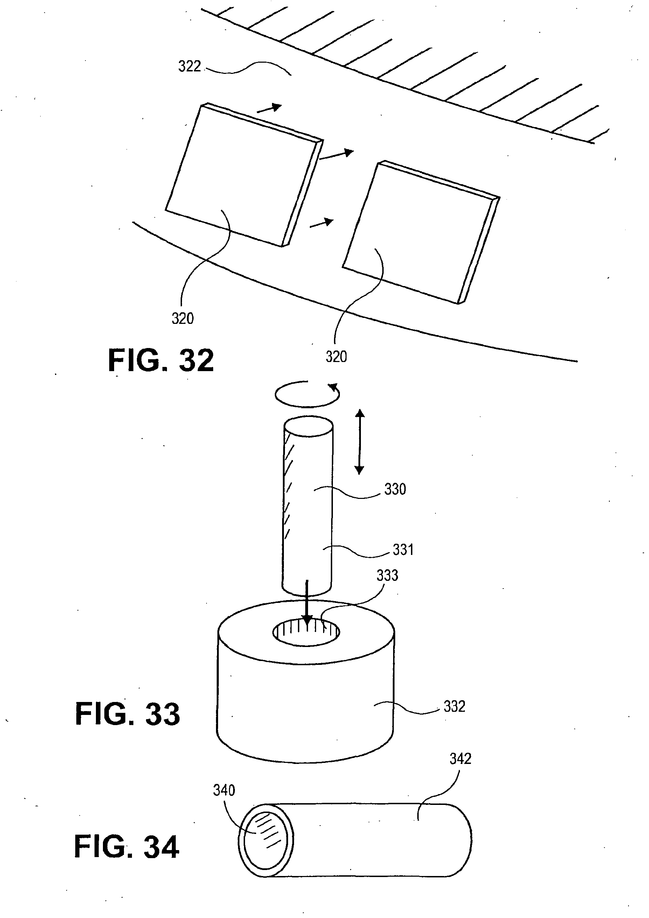

The invention has non-medical applications. For example, Figure 32 shows the use of a lubricious IPN or semi-IPN composition of this invention to resurface the hull of a marine vessel. Panels 320 of a thermoplastic gradient IPN (as described above) have been attached to the surface of hull 322 to reduce drag and biofilm formation. Alternatively, the IPN material can be in some embodiments painted on the hulls as a liquid and allowed to cure or harden. The gradient IPN can be negatively charged on its surface or uncharged and can be made from one or more types of monomer species. Various UV protection and anti-oxidizing agents or other additives can also be incorporated into these materials to improve their performance.

Figure 33 shows the use of a lubricious thermoplastic or thermoset IPN (as described above) to modify interfacing surfaces of machine parts that move with respect to each other, such as surface 331 of rotating and translating part 330 and surface 333 of stationary part 332. Figure 34 shows the use of a lubricious thermoplastic or thermoset IPN (as described above) to reduce fluid drag on the inner surface 340 of a pipe 342.

The materials of the present disclosure have utility in applications requiring electrochemical conductivity. The conductivity of the IPNs and semi-IPNs is based on the flow of ions through the hydrated matrix of the material. Thin films of polyetherurethane were swelled with four different compositions of an acrylic acid and water mixture (15, 30, 50, and 70% acrylic acid in water). Each swelled film was then cured in UV light to form the semi-IPN. The films were then neutralized in PBS. The electrical resistance of the materials was measured using an ohm meter. To measure resistance, the IPN film was lightly patted with a paper towel to remove excess PBS and the ohm meter probes were clipped to the film across a film width of 60-70 mm. The initial and steady-state resistance values were recorded. In addition, the resistances of an unmodified polyetherurethane film and liquid PBS were measured. The resistance of PBS was measured by placing the ohm meter probes directly into a PBS bath at an approximate distance of 60 mm between the probes. Resistance measurements are in the following Table.

**Table 1**

| **Material** | **Lowest resistance reading (kΩ)** | **Steady-state resistance reading (kΩ)** |
|---|---|---|
| PEU alone (0% AA) | out of range (dielectric) | out of range (dielectric) |
| PEU/PAA (15% AA) | 175 | 200 |
| PEU/PAA (30% AA) | 132 | 177 |
| PEU/PAA (50% AA) | 150 | 161 |
| PEU/PAA (70% AA) | 110 | 141 |
| PBS bath | 300 | 600 |

The results show that the resistances of the semi-IPNs are lower than (but within the same order of magnitude as) pure PBS fluid alone. The limit of the ohm meter was 40,000 ohms. Typical values for insulators (including polyurethanes) are 10¹⁴- 10¹⁶ ohms; therefore, the resistance values of the PEU alone were outside the range of the meter used. Permeability of the PEU/PAA semi-IPN was measured using a device similar to the one described by Maroudas et al. in Permeability of articular cartilage. Nature, 1968. 219(5160): p. 1260-1. The permeability was calculated according to Darcy's Law (Q = KAΔp/L), where Q is the flow rate [mm³/sec], A the cross-sectional area of the plug [mm²], *Δp* the pressure gradient applied [MPa] (pressurized fluid), *L* is the thickness of the hydrogel. The permeability of the PEU/PAA semi-IPN prepared from 70% acrylic acid was found to be *K* = 1.45×10⁻¹⁷ m⁴/N*sec. For natural cartilage, literature values range from 1.5 × 10⁻¹⁶ to 2×10⁻¹⁵ m⁴/N*sec. Therefore, the PEU/PAA is 10-100 times less permeable than cartilage, which may make it less prone to dehydration under prolonged compressive loads compared to natural cartilage. The permeability of the IPN can be tuned by varying the concentration of AA in the swelling solution; the higher the AA content, the higher the permeability. In contrast, the unmodified PEU material alone is effectively impermeable to solutes; although it retains some moisture (~ 1%), in practice it does not act as a solute-permeable matrix.

Other variations and modifications to the above compositions, articles and methods include:

The first polymer can be one that is available commercially or custom-made and made by a number of ways (e.g., extruded, injection molded, compression molded, reaction injection molded (RIM) or solution-casted.) The first polymer can be uncrosslinked or crosslinked by various means. Either polymer can be crosslinked by, e.g., gamma radiation or electron beam radiation.

Any number or combinations of ethylenically unsaturated monomers or macromonomers (e.g., containing reactive double bonds) can be used as the basis of the second or subsequent network so long as the total contains at least 2% by weight ionizable chemical groups. These include but are not limited those containing vinyl, acrylate, methacrylate, allyl ether, or acrylamide groups. And number of pendant functional groups can be conjugated to these ethylenicaly unsaturated groups including but not limited to carboxylic acid, sulfonic acid, acetates, alcohols, ethers, phenols, aromatic groups, or carbon chains.

The polyurethane-based polymer can be (but is not limited to) the following: polyether urethane, polycarbonate urethane, polyurethane urea, silicone polyether urethane, or silicone polycarbonate urethane. Other polyurethanes with other hard segments, soft segments, and chain extenders are possible.

Other polymers can be used in the first network, such as homopolymers or copolymers of silicone (polydimethylsiloxane) or polyethylene.

When a polyurethane-based polymer is used as the first polymer, the extent of physical and chemical crosslinking of the polyurethane-based polymer can be varied between physical crosslinking-only (thermoplastic) to extensive chemical crosslinking. In the case of chemical crosslinking, the crosslinkable polyurethane can be used alone or as a mixture with thermoplastic (uncrosslinked) polyurethane.

The conditions of polymerization (i.e., ambient oxygen, UV intensity, UV wavelength, exposure time, temperature) may be varied.

The orientation and steepness of the composition gradients can be varied by various means such as time and/or method of immersion in the monomer, and the application of external hydrostatic positive or negative pressure.

The thermoplastic can be made porous by various techniques such as foaming or salt-leaching. After swelling of the porous polymer (such as PU) with a monomer (such as AA) followed by polymerization or AA, a porous IPN is formed.

Additional layers of thermoplastics can be added to material on either the IPN side or the thermoplastic side-only by curing or drying the new thermoplastic to the surface. The layers can all be the same material or be different materials (e.g. ABS + polyurethane, polyether urethane + polycarbonate urethane).

A number of different solvents can be used during the synthesis of the polyurethane, the second network, or both, including but not limited to dimethylacetamide, tetrahydrofuran, dimethylformamide, ethanol, methanol, acetone, water, dichloromethane, propanol, methanol, or combinations thereof.

Any number of initiators can be used such as photoinitiators (e.g., phenone-containing compounds and lrgacure® products), thermal initiators, or chemical initiators. Examples of thermal initiators include but are not limited to azo-compounds, peroxides (e.g., benzoyl peroxide), persulfates (e.g., potassium persulfate or ammonium persulfate), derivatives, or combinations thereof.

Variations of the crosslinking identity and density (e.g. 0.0001% - 25% by mole crosslinking agent with respect to the monomer), initiator concentration (e.g. 0.0001% - 10% by mole with respect to the monomer) molecular weight of precursor polymers, relative weight percent of polymers, light wavelength (UV to visible range), light intensity (0.01 mW/cm² - 1 W/cm²), temperature, pH and ionic strength of swelling liquid, and the level of hydration.

The second network material can be synthesized in the absence of a crosslinking agent.

The water content of these materials can range between 2% to 99%.

Different components of the IPN can be incorporated in combination with ionizable monomers, such as poly(vinyl alcohol), poly(ethylene glycol)-acrylate, poly(2-hydroxyethylacrylate), poly(2-hydroxyethylmethacrylate), poly(methacrylic acid), poly(2-acrylamido-2-methyl propane sulfonic acid), other vinyl-group containing sulfonic acids, poly(acrylamide), poly(N-isopropylacrylamide) poly(dimethacrylamide), and combinations or derivatives thereof. For instance, a copolymer of acrylic acid and vinyl sulfonic acid or 2-acrylamido-2-methyl propane sulfonic acid can be created for the second network to form a polyurethane first network and a poly(acrylic acid-co-acrylamido-methylpropane sulfonic acid) copolymeric second network. Any monomer or combination of monomers can be used in conjunction with a suitable solvent as long as they contain at least 2% by weight ionizable monomer and are able to enter (swell) the first polymer.

The IPN can have incorporated either chemically or physically within its bulk or its surface certain additives such as antioxidants (e.g., Vitamin C, Vitamin E, Irganox®, or santowhite powder) and/or anti-microbial agents (e.g., antibiotics). These can be chemically linked to the material by, for example, esterification of the antioxidant with any vinyl-group containing monomer such as methacrylate, acrylate, acrylamide, vinyl, or allyl ether.

More than two networks (e.g., three or more) can also be formed, each of which are either crosslinked or uncrosslinked.

The polyurethane itself can be modified in a number of ways, such as by sulfonation at the urethane group by reaction of 1,3 propane sulfone in the presence of sodium hydride, or the formation of allophanate linkages at the urethane group by reaction with excess isocyanate groups. For instance, excess isocyanatoethyl methacrylate can be reacted with polyurethane in toluene in the presence of dibutyltin dilaurate for 2.5 hours to yield a methacryloxy-conjugated polyurethane surface. The methacryloxy groups can then be used subsequently tether other methacryloxy (or other vinyl group)-containing monomers or macromonomers via free radical polymerization. Such modifications can be carried out before or after the formation of the second network of the IPN.

Other modifications will be apparent to those skilled in the art.

### EXAMPLES

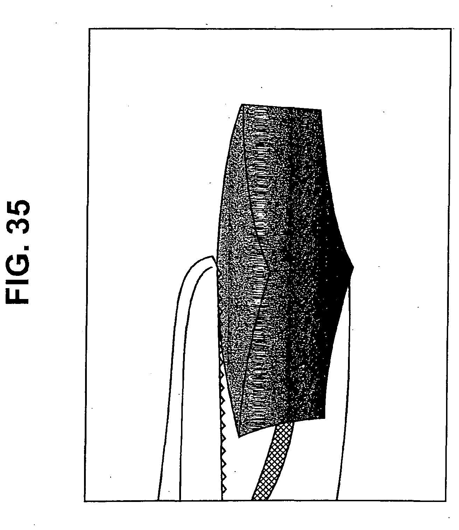

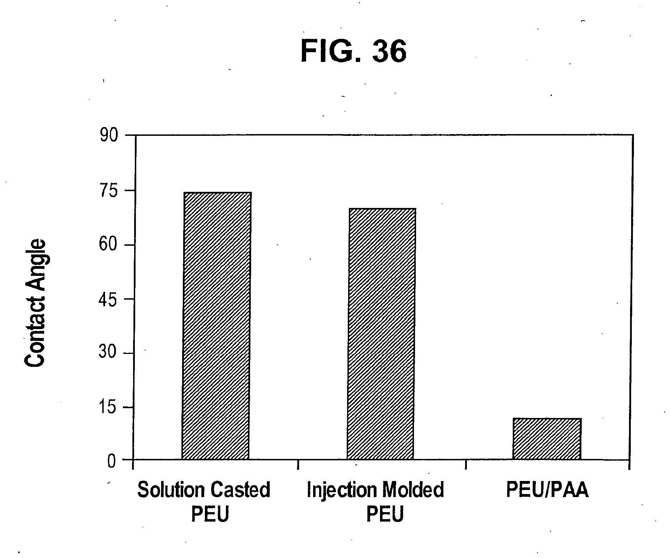

Examples 26 and 27 are according to the present invention. All other examples are described for reference only.
**Example 1** In one example, a polycarbonate urethane (Bionate 55D) was immersed in 70% acrylic acid in water containing 0.1% v/v 2-hydroxy-2-methyl propiophenone and 0.1% v/v triethylene glycol dimethacrylate with respect to the monomer overnight. The polycarbonate urethane was removed from the solution, placed between two glass slides, and exposed to UV light (2 mW/cm²) for 15 minutes. The resulting semi-IPN was removed, and washed and swollen in phosphate buffered saline. The material swelled and became lubricious within hours. In other examples, segmented polyurethane urea, as well as silicone polyether urethane and silicone polycarbonate urethanes were placed in acrylic acid solutions and polymerized and washed in the same fashion to yield a lubricious IPN.
**Example 2** In another example, a polyether urethane (Elasthane™ 55D) was immersed in 70% acrylic acid in water containing 0.1% v/v 2-hydroxy-2-methyl propiophenone and 0.1% v/v triethylene glycol dimethacrylate with respect to the monomer overnight. The polyether urethane was removed from the solution, placed between two glass slides, and then exposed to UV light (2 mW/cm²) for 15 minutes. The resulting semi-IPN was removed and then washed and swollen in phosphate buffered saline. The material swelled and became lubricious within hours. In other examples, polycarbonate urethane, segmented polyurethane urea, as well as silicone polyether urethane and silicone polycarbonate urethanes were placed in acrylic acid solutions and polymerized and washed in the same fashion to yield lubricious IPNs.
**Example 3** In another example, silicone polyether urethane and silicone polycarbonate urethanes were separately placed overnight in 100% acrylic acid solutions, to which were added 0.1% v/v 2-hydroxy-2-methyl propiophenone and 0.1% v/v triethylene glycol dimethacrylate with respect to the monomer. After polymerization and crosslinking, the semi-IPNs swelled and became lubricious. The addition of silicone (polydimethylsiloxane) in the polyurethane adds an extra level of biostability to the material as well as potentially useful surface chemistry and properties.
**Example 4** In another example, a methacryloxy-functionalized polycarbonate urethane was exposed to UV light to crosslink the polycarbonate urethane, and then swollen in 70% acrylic acid with 0.1% v/v 2-hydroxy-2-methyl propiophenone and 0.1% v/v triethylene glycol dimethacrylate with respect to the monomer overnight. The material was removed from the solution, placed between two glass slides, and then exposed to UV light (2 mW/cm²) for 15 minutes to yield a fully interpenetrating polymer network of the polycarbonate urethane and poly(acrylic acid.) The IPN was then washed in an aqueous salt solution to neutralize the poly(acrylic acid), achieve equilibrium swelling, and remove any unreacted monomers.
**Example 5** In another example, a methacryloxy-functionalized polyether urethane was exposed to UV light (in the presence of 0.1% 2-hydroxy-2-methyl propiophenone and 0.1% triethylene glycol dimethacrylate) to crosslink the polyetherurethane, and then was swollen in 70% acrylic acid with the aforementioned photoinitiator and crosslinker followed by UV-initiated crosslinking to yield a fully interpenetrating polymer network of the polyetherurethane and poly(acrylic acid.) The IPN was then washed in an aqueous salt solution to neutralize the poly(acrylic acid), achieve equilibrium swelling, and remove any unreacted monomers.
**Example 6** In another example, a 25% solution of methacryloxy-functionalized polycarbonate urethane in DMAC along with 0.1% of the aforementioned photoinitiator was exposed to UV light to crosslink the polycarbonate urethane. After removing the solvent in a heated (60° C) convection oven, an additional layer of polycarbonate urethane was then cast on one side of the crosslinked polycarbonate urethane to yield a laminate structure and then only the crosslinked side was swollen in 70% acrylic acid with the 0.1% 2-hydroxy-2-methyl propiophenone and 0.1% triethylene glycol dimethacrylate followed by UV-initiated crosslinking to yield a fully interpenetrating polymer network of the polycarbonate urethane and poly(acrylic acid.) The IPN was then washed in an aqueous salt solution to neutralize the poly(acrylic acid), achieve equilibrium swelling, and remove any unreacted monomers.
**Example 7** In another example, a 25% solution of methacryloxy-functionalized polycarbonate urethane in DMAC along with 0.1% of the aforementioned photoinitiator was exposed to UV light to crosslink the polyether urethane. After removing the solvent in a heated (60° C) convection oven, an additional layer of polyether urethane was then cast on one side of the crosslinked polycarbonate urethane to yield a laminate structure and then only the crosslinked side was swollen in 70% acrylic acid with 0.1% 2-hydroxy-2-methyl propiophenone and 0.1% triethylene glycol dimethacrylate followed by UV-initiated crosslinking to yield a fully interpenetrating polymer network of the polyether urethane and poly(acrylic acid.) The IPN was then washed in an aqueous salt solution to neutralize the poly(acrylic acid), achieve equilibrium swelling, and remove any unreacted monomers.
**Example 8** In another set of examples, a layer of methacroxy-functionalized polyether urethane was cast onto a layer of injection molded polyether urethane, and separately, another layer was cast onto a layer of injection molded polycarbonate urethane. Each was exposed to UV light, to yield laminate structures. Only the crosslinked sides were swollen in 70% acrylic acid with 0.1% 2-hydroxy-2-methyl propiophenone and 0.1% triethylene glycol dimethacrylate followed by UV-initiated crosslinking to yield a fully interpenetrating polymer networks. The IPNs were then washed in an aqueous salt solution to neutralize the poly(acrylic acid), achieve equilibrium swelling, and remove any unreacted monomers.
**Example 9** In one example, acrylonitrile butadiene styrene (ABS) was exposed to 100% acrylic acid in water containing 0.1% v/v 2-hydroxy-2-methyl propiophenone and 0.1% v/v triethylene glycol dimethacrylate with respect to the monomer for 15 minutes. The surface-exposure was accomplished by drop-casting the monomer solution on the surface of the ABS for 30 minutes. The ABS was then placed between two glass slides, and then exposed to UV light (2 mW/cm²) for 15 minutes. The resulting ABS/PAA gradient IPN was removed and then washed and swollen in phosphate buffered saline. The IPN was washed in an aqueous salt solution to neutralize the poly(acrylic acid), achieve equilibrium swelling, and remove any unreacted monomers. The material swelled and became lubricious within hours.
**Example 10** To reshape the thermoplastic gradient IPNs, heat was applied. An ABS/PAA gradient IPN was heated using a heat gun and then laid on a cylindrical polypropylene tube. After letting the material cool to room temperature, acetone was injected between the ABS/PAA and the polypropylene. After applying manual pressure and allowing the sample to dry, the result was a thermoplastic gradient IPN wrapped around and bonded to a polypropylene tube.
**Example 11** In another example, a thermoplastic gradient ABS/PAA IPN was attached to polycarbonate urethane by injecting acetone between the ABS and polycarbonateurethane and applying manual pressure to yield a thermoplastic gradient IPN bonded to a polycarbourethane.
**Example 12** In another example, a curved polycarbonate urethane IPN was made straight again by applying heat on the polyurethane side using a heat gun, manually reversing the curvature of the material, and cooling the IPN in water.
**Example 13** In another example, a polyether urethane solution (e.g. 20% in dimethylacetamide ("DMAC")) was cast on top of a polycarbonate urethane in a laminate structure, allowed to dry in a heated (60° C) convection oven, and then only the polyether urethane surface was exposed to 70% acrylic acid in water containing 0.1% v/v 2-hydroxy-2-methyl propiophenone and 0.1% v/v triethylene glycol dimethacrylate with respect to the monomer for 15 minutes. The surface-exposure was accomplished by laying the laminate material polyether urethane-side down on a bed of fabric that was soaked in the aforementioned monomer solution. The material was removed from the fabric mat, placed between two glass slides, and then exposed to UV light (2 mW/cm²) for 15 minutes. The resulting gradient semi-IPN was removed, washed and swollen in phosphate buffered saline. The material swelled and became lubricious within hours. In other examples, polyether urethane, segmented polyurethane urea, silicone polyether urethane, and silicone polycarbonate urethane were handled the same way to yield a lubricious semi-IPNs.
**Example 14** In another example, a layer of polycarbonate urethane (20% in DMAC) containing 50% by weight sodium chloride was solution cast on a premade polyether urethane-polycarbonate urethane and dried at 80°C under convection. The salt was washed away in water to yield a porous side on the laminated polyurethane. Other materials have been made with sodium chloride concentrations varying between 10% and 80%
**Example 15** In another example, a layer of polycarbonate urethane (20% in DMAC) containing 20% tricalcium phosphate was solution cast on a premade polyether urethane-polycarbonate urethane and dried at 80°C under convection. The tricalcium phosphate was left embedded within the polyurethane as an osteoconductive agent. Other materials have been made with tricalcium phosphate concentrations varying from 0.001% - 20%
**Example 16** In another example, a polyurethane urea (e.g. 20% in dimethylacetamide) was cast on top of a polycarbonate urethane in a laminate structure, and then only the polyurethane urea surface was exposed to 70% acrylic acid in water containing 0.1% v/v 2-hydroxy-2-methyl propiophenone and 0.1% v/v triethylene glycol dimethacrylate with respect to the monomer for 15 minutes. The surface-exposure was accomplished by laying the laminate material polyurethane urea-side down on a bed of fabric that was soaked in the aforementioned monomer solution. The polycarbonate urethane was removed from the fabric mat, placed between two glass slides, and then exposed to UV light (2 mW/cm²) for 15 minutes. The resulting gradient semi-IPN was removed and then washed and swollen in phosphate buffered saline. The material swelled and became lubricious within hours. The material was washed in PBS to neutralize the poly(acrylic acid), achieve equilibrium swelling, and remove any unreacted monomers.
**Example 17** In another example, a methacryloxy-functionalized polyether urethane mixed with a thermoplastic polyether urethane in solution (25% in dimethylacetamide) was exposed to UV light to crosslink the polycarbonate urethane. An additional layer of polyether urethane was then cast on one side of the crosslinked polyether urethane to yield a laminate structure and then only the crosslinked side was swollen in 70% acrylic acid with the aforementioned photoinitiator and crosslinker, followed by UV-initiated crosslinking to yield a fully interpenetrating polymer network of the polyether urethane and poly(acrylic acid.) The IPN was then washed in an aqueous salt solution to neutralize the poly(acrylic acid), achieve equilibrium swelling, and remove any unreacted monomers.
**Example 18** In one example, flat sheets were created by solution casting of thermoplastic polyurethanes in (dimethylacetamide (DMAC). Polyurethane solutions of polyether urethane (Elasthane™), polycarbonate urethane (Bionate), polyether urethane urea (Biospan), silicone polycarbonate urethane (Carbosil), and silicone polyether urethane (Pursil) were synthesized in dimethylacetamide (DMAC) at solids concentrations of about 25% by the manufacturer.
**Example 19** Spherical shapes were cast by dip-coating glass as well as silicone spheres in polyurethane solutions (in DMAC). Polycarbonate urethane (20% in DMAC) was dip coated onto a spherical glass mold (49.5 mm outer diameter), and separately, onto a silicone sphere. The solvent was removed by drying at 80°C in a convection oven. This process was repeated two more times to create three total coatings. Then, the sphere was dip coated in polyether urethane (20% in DMAC) and then dried at 80°C under convection. This process was also repeated two more times. The resulting capped-shaped, laminate polyurethane was removed from the mold, and its outer side immersed in a 70% acrylic acid solution in water, with 0.1% 2-hydroxy-2-methyl-propiophenone and 0.1% triethylene glycol dimethacrylate for 1.5 hours. The cap was inverted, placed back over a spherical glass mold, and exposed to UV light (2 mW/cm²) for 15 minutes. Next the cap was removed from the mold and placed in phosphate buffered saline. The result was a spherical, gradient IPN with one lubricious surface and one pure thermoplastic surface. Other temperatures and other solvents can also be used to carry out this process, as well as other mold materials and polymer components.
**Example 20** In another example, a polyether urethane was swollen in 70% acrylic acid with 0.1% 2-hydroxy-2-methyl propiophenone and 0.1% methylene bisacrylamide. One side of the material was dabbed dry, and then exposed to air and treated with UV light. The resulting gradient semi-IPN was then washed in an aqueous salt solution to neutralize the poly(acrylic acid), achieve equilibrium swelling, and remove any unreacted monomers. In other experiments, the material was exposed to nitrogen or argon during curing.
**Example 21** In another example, a polyether urethane (Elasthane™ 55D) was injection molded and then swollen in 70% acrylic acid with 0.1% v/v 2-hydroxy-2-methyl propiophenone and 0.1% w/w methylene bisacrylamide followed by UV-initiated crosslinking to yield a fully interpenetrating polymer network of the polyether urethane and poly(acrylic acid). The IPN was then washed in an aqueous salt solution to neutralize the poly(acrylic acid), achieve equilibrium swelling, and remove any unreacted monomers.
**Example 22** In another example, a polyether urethane (Elasthane™ 75D) was injection molded, dip-casted (solution casted) on one side in a polyether urethane solution (Elasthane™ 55D in 25% DMAC) and dried in a convection oven to remove the DMAC solvent. The dried material was swollen in 70% acrylic acid with the 70% acrylic acid with 0.1% v/v 2-hydroxy-2-methyl propiophenone and 0.1% w/w methylene bisacrylamide followed by UV-initiated crosslinking to yield a fully interpenetrating polymer network of the polyether urethane and poly(acrylic acid). The IPN was then washed in an aqueous salt solution to neutralize the poly(acrylic acid), achieve equilibrium swelling, and remove any unreacted monomers.
**Example 23** In another example, a polycarbonate urethane (Bionate 75D) was injection molded, dip-casted (solution casted) on one side in a polyether urethane solution (Elasthane™ 55D in 25% DMAC) and dried in a convection oven to remove the DMAC solvent. The dried material was swollen in 70% acrylic acid with 0.1% v/v 2-hydroxy-2-methyl propiophenone and 0.1% w/w methylene bisacrylamide followed by UV-initiated crosslinking to yield a fully interpenetrating polymer network of the polyether urethane and poly(acrylic acid). The IPN was then washed in an aqueous salt solution to neutralize the poly(acrylic acid), achieve equilibrium swelling, and remove any unreacted monomers.
**Example 24** In another example, a polyether urethane (Elasthane™ 75D) was injection molded and then dip-casted (solution casted) in a methacryloxy-functionalized polyether urethane solution (Elasthane™ 55D in 25% DMAC) along with the aforementioned photoinitiator and then was exposed to UV light to crosslink the methacryloxy-functionalized polyether urethane. The material was then dried in a convection oven to remove the DMAC solvent. The dried material was then swollen in 70% acrylic acid with the 0.1% v/v 2-hydroxy-2-methyl propiophenone and 0.1% w/w methylene bisacrylamide followed by UV-initiated crosslinking to yield a fully interpenetrating polymer network of the polyether urethane and poly(acrylic acid). The IPN was then washed in an aqueous salt solution to neutralize the poly(acrylic acid), achieve equilibrium swelling, and remove any unreacted monomers.
**Example 25** In another example, a polycarbonate urethane (Bionate 75D) was injection molded and then dip-casted (solution casted) in a methacryloxy-functionalized polyether urethane solution (Elasthane™ 55D in 25% DMAC) and then was exposed to UV light to crosslink the methacryloxy-functionalized polyether urethane. The material was then dried in a convection oven to remove the DMAC solvent. The dried material was then swollen in 70% acrylic acid with the 0.1% v/v 2-hydroxy-2-methyl propiophenone and 0.1% v/v triethylene glycol dimethacrylate followed by UV-initiated crosslinking to yield a fully interpenetrating polymer network of the polyether urethane and poly(acrylic acid). The IPN was then washed in an aqueous salt solution to neutralize the poly(acrylic acid), achieve equilibrium swelling, and remove any unreacted monomers.
**Example 26** In another example, a polyether urethane (Elasthane™ 55D) solution casted and then swollen in 35% sulfopropyl methacrylate in acetic acid with 0.1% v/v 2-hydroxy-2-methyl propiophenone and 0.1% w/w methylene bisacrylamide followed by UV-initiated crosslinking to yield a fully interpenetrating polymer network of the polyether urethane and poly(acrylic acid). The semi-IPN was then washed with water to remove the acetic acid, and then in an aqueous salt solution to neutralize the poly(acrylic acid), achieve equilibrium swelling, and remove any unreacted monomers.
**Example 27** In another example, a polyether urethane (Elasthane™ 55D) solution casted and then swollen in 35% sulfopropyl methacrylate and 35% acrylic acid in water with the 0.1% v/v 2-hydroxy-2-methyl propiophenone and 0.1% w/w methylene bisacrylamide followed by UV-initiated crosslinking to yield a fully interpenetrating polymer network of the polyether urethane and poly(acrylic acid). The semi-IPN was then washed in an aqueous salt solution to neutralize the poly(acrylic acid)/poly(sulfopropyl methacrylate) copolymer, achieve equilibrium swelling, and remove any unreacted monomers.
**Example 28** In another example, a rectangular sample of PMMA (plexiglass) was swollen briefly in 100% acrylic acid in water with the 0.1% v/v 2-hydroxy-2-methyl propiophenone and 0.1% w/w methylene bisacrylamide followed by UV-initiated crosslinking to yield a fully interpenetrating polymer network of the PMMA and poly(acrylic acid). The IPN was then washed in an aqueous salt solution to neutralize the poly(acrylic acid), achieve equilibrium swelling, and remove any unreacted monomers.
**Example 29** In another example, a rectangular specimen of polydimethyl sulfoxide (PDMS, Sylgard® 184) was prepared according to the manufacturer's specifications and then was swollen briefly in a 35% acrylic acid solution in tetrahydrofuran along with 0.1% v/v 2-hydroxy-2-methyl propiophenone and 0.1% v/v triethylene glycol dimethacrylate, followed by UV-initiated crosslinking to yield a fully interpenetrating polymer network of the PDMS and poly(acrylic acid). The IPN was washed in an aqueous salt solution to neutralize the poly(acrylic acid), achieve equilibrium swelling, and remove any unreacted monomers.
**Example 30** Figure 35 is a cross-section of a hydrated arthroplasty device and shows that the arthroplasty device is, in effect, a synthetic version of an osteochondral graft that emulates the structure, elastic modulus, fracture strength, and lubricious surface of natural cartilage on one side and the stiffness, strength, and porosity of trabecular bone on the other side. The device is comprised of a composite gradient material featuring a lubricious, cartilage-like polymer that smoothly transitions into a stiff, porous, bone-like anchoring surface. The gradient was designed to mimic the compositional gradient inherent to natural joints, in which compliant, slippery cartilage becomes progressively more hard and bone-like from superficial to deep along the thickness direction. In practice, this "biomimetic" gradient should yield a physiologic stress distribution over the underlying bone while also minimizing micromotion at the bone interface by effectively matching the stiffnesses of the device and bone at their point of contact. Suitable materials are described, e.g., in the following: US Patent Appl. SN 61/079,060 (filed 7/8/2008); US Patent Appl. SN 61/095,273 (filed 9/8/2008); and US Patent Appl. SN 12/148,534 (filed 4/17/2008).
**Example 31** Figure 36 shows contact angle analysis indicating that the material of this disclosure is very hydrophilic. When a drop of water is placed on a surface, the shape the drop takes is dependent on the composition of the surface. A hydrophilic surface attracts the water and creates a flatter drop, while a hydrophobic surface repels the water and creates a rounder drop. The degree of hydrophilicity of the surface is inferred by measuring the angle created between the surface and the drop of water, referred to as the contact angle. Typically, a more hydrophilic surface will have a contact angle of about 0-45° with water, while a more hydrophobic surface will have a contact angle greater than 45° with water.

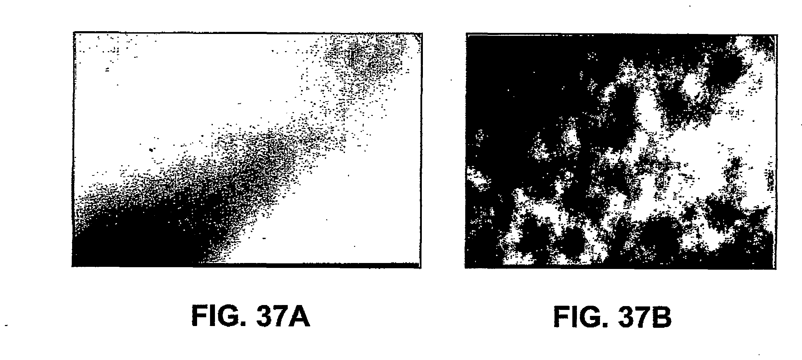

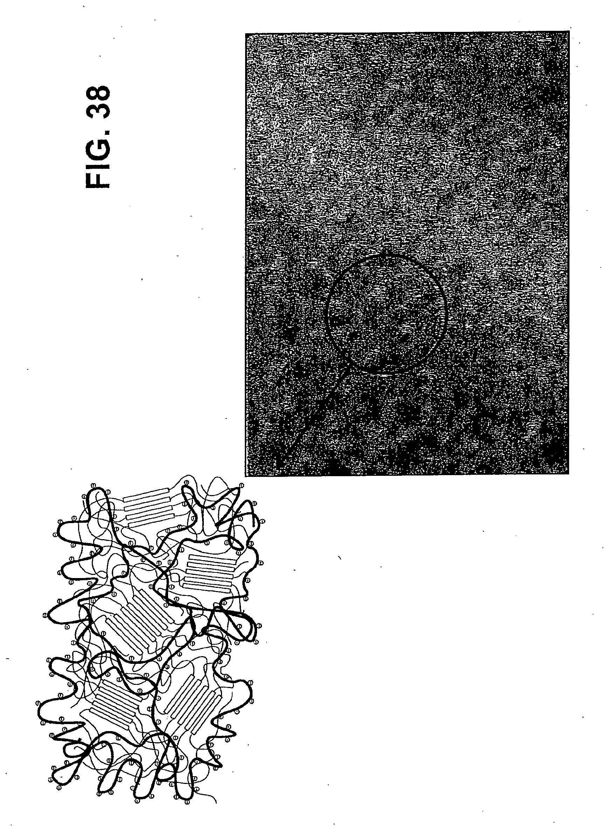

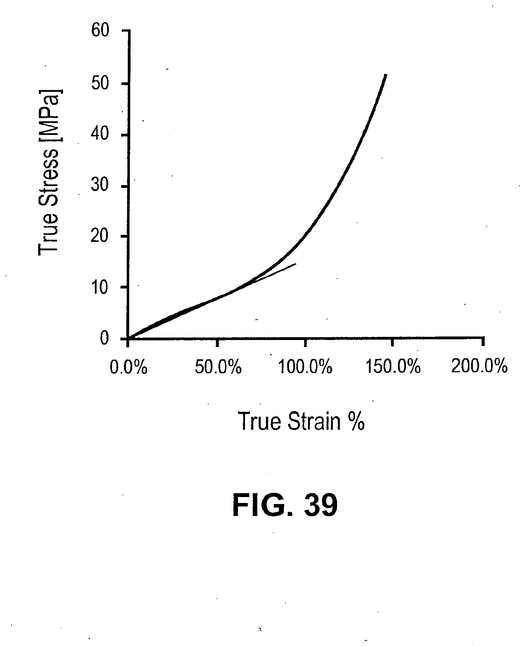

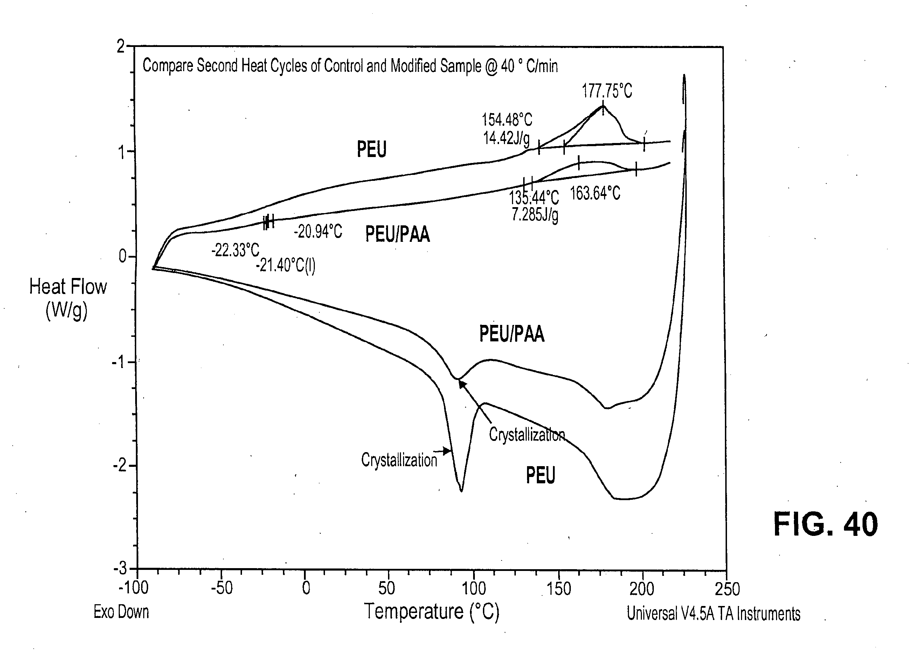

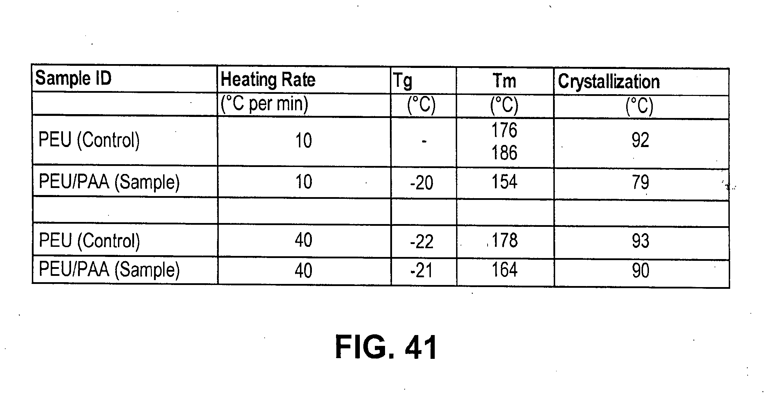

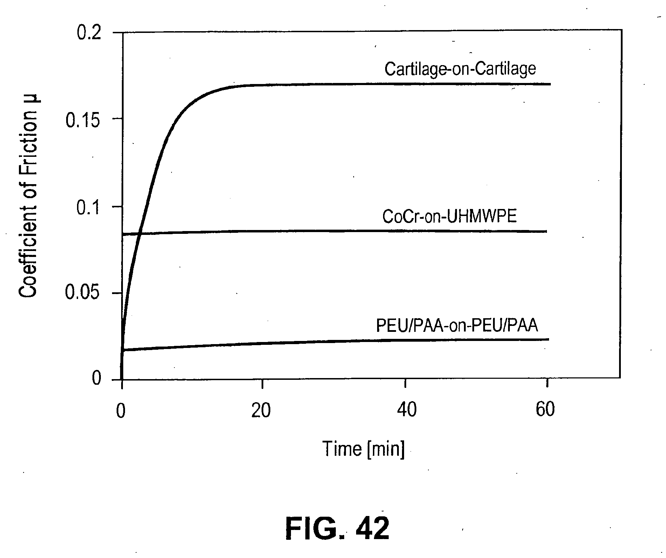

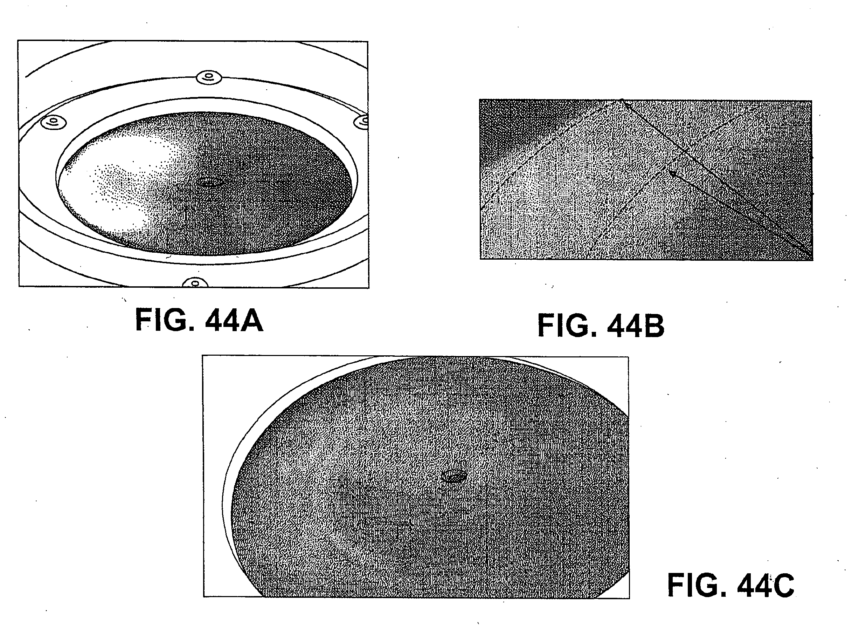

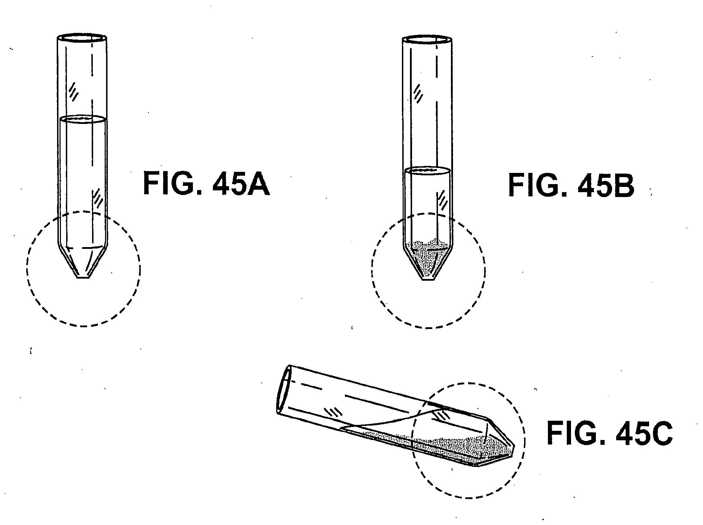

The contact angle between the charged hydrogel IPN made according to this disclosure and water was determined. Briefly, a sheet of Elasthane™ 55D (polyetherurethane) was soaked in acrylic acid with initiator and cross-linker, and cured to form a semi- IPN (PEU/PAA semi IPN). After curing, the charged PEU/PAA semi IPN was hydrated in phosphate buffered saline. The material was removed from the solution and its surface briefly dabbed to remove any residual liquid. A drop of water was placed on the surface of the material, and the contact angle read using a Goniometer. The results showed a contact angle of approximately 8°. For comparison, readings taken on starting materials of solution-casted polyurethanes and injection-molded polyurethane had contact angles of approximately 72° and 69°, respectively. This result demonstrates that the incorporation of a poly(acrylic acid) network into polyurethane according to the current disclosure dramatically increases surface hydrophilicity.
**Example 32** The differences in the structures of the charged hydrogel IPN and polyurethane are shown by Transmission Electron Microscopy (TEM). TEM creates a highly magnified image of a material. TEM was performed on samples of polyetherurethane/poly(acrylic) acid semi IPN (PEU/PAA semi IPN) of the current disclosure and of unmodified polyetherurethane. Briefly, a sheet of Elasthane™ 55D (polyetherurethane) was soaked in acrylic acid with initiator and cross-linker, and cured. It was stained with osmium tetroxide per standard procedures to perform TEM analysis. Figure 37A shows a 34kX magnification image of PEU while Figure 37 B shows the PEU/PAA semi-IPN. The sizes of light and dark regions, corresponding to the amorphous (soft) and ordered (hard) domains, are increased in the TEM images of the PEU/PAA semi-IPN relative to the unmodified PEU. The PAA appears sequestered within the PEU soft segments. on the basis of the larger domain sizes in the PEU/PAA sample compared to the PEU sample, the degree of phase separation is greater in the PEU/PAA sample compared to the unmodified PEU.
**Example 33** Figure 38 shows a TEM of the same PEU/PAA semi-IPN material as Figure 37 at 12.4 kX magnification. The schematic illustrates how the hard segments are phase separated from the soft segments of the interpenetrated polymer network.
**Example 34** Figure 39 shows the static mechanical properties of the PEU/PAA IPN which comprises an exemplary joint interface surface of an orthopaedic implant. Uniaxial tensile tests were conducted to determine the initial Young's modulus in tension, the strain-at-break, and stress-at-break of the materials. Dog bone specimens were tested according to ASTM D638, at a strain rate of 0.3%/sec. The average true stress - true strain curve for the material of the joint interface material is presented in Figure 40. In the linear portion of the curve, the elastic modulus (as provided from the true stress, true strain curve) is E = 15.3 MPa which is very close to the tensile properties reported for natural cartilage. The ultimate true stress was found to be at approximately σᵤₗₜ = 52 MPa at εᵤₗₜ = 143% true strain (of note, cartilage is found to fail at around 65% strain). Strain hardening under tension was observed for true strains of 80% and higher. The Poisson's ratio (equilibrium) was estimated by measuring the lateral contraction of the dog bone neck region and was found to be consistent along the strain range at v=0.32. The bulk modulus was therefore calculated from the equation K = E/3(1-2v) and was found to be 18.3 MPa. Unconfined compression plug tests according to ASTM D695 reveal that PEU/PAA semi-IPN has excellent compressive properties, with a compressive stiffness modulus of 15.6 MPa (same as the tensile modulus, based on true stress-strain) and a failure strength that is higher than 50 MPa.
**Example 35** Figures 40 shows the thermal curves of PEU and PEU/PAA semi-IPN samples evaluated by Differential Scanning Calorimetry (DSC) at a heating rate of 40° C per minute. Figure 41 compares the thermal transitions of PEU and PEU/PAA semi-IPN samples evaluated by DSC at two different heating rates. The thermal transition temperatures including the glass transition temperature T_{g}, the crystallization temperature, and the melting temperature Tm were determined. Below its T_{g}, the heat capacity of the polymer is lower and the polymer is harder or glassier. Above the T_{g}, the heat capacity of the polymer increases and the polymer becomes more flexible. Above this temperature, for some polymers is the crystallization temperature and at least some of the domains of the molecule become more organized, and essentially crystalline. At a higher temperature is the melting temperature when the crystalline portions completely melt. The procedure was done following ASTM D3418-03 test method using a TA Instruments Q200 DSC system with a Modulated Differential Scanning Calorimeter and Refrigerated Cooling System (RCS90). Briefly, a sheet of Elasthane™ 55D (polyetherurethane) was soaked in acrylic acid with an initiator and cross-linker and then cured. A small amount (2-6 mg) of PEU/PAA semi-IPN sample was placed into a first aluminum pan. A cover was placed on the top of the pan and crimped with a Universal Crimping press to sandwich the sample between pan and cover. Heat was applied to the first pan and, separately, to a reference pan, and the current flow to each was changed to keep the temperatures of the two materials the same. The heat flow of the material being tested was graphed against the temperature and the slopes of the curves indicate the thermal transition temperatures (Figure 40). Several tests were performed, using different rates of heating (10°C and 40°C per minute). By performing the tests at different rates of heating, different resolution is obtained for the thermal transitions, as seen in Figure 41. Because the T_{g} can depend on the previous thermal history of the material, the material is subjected to two heat cycles. The first heat cycle is used to standardize the conditions under which the polymer arrives at its test state, and the second test cycle is used to generate transition temperatures. The glass transition temperatures, T_{g}, for both the PEU/PAA semi IPN and the PEU were around 21°C when the rate of heating was kept at 10°C per minute. The crystallization and melting temperatures were lower in the PEU/PAA compared with the PEU. At a heating rate of 40°C per minute, the crystallization temperatures were 90°C for the PEU/PAA compared with 93°C for the PEU. When the heating rate was slowed to 10°C per minute, the crystallization temperatures observed were 79°C for the PEU/PAA compared with 92°C for the PEU. Finally, at a heating rate of 40°C per minute, the Tm temperatures were 164°C for the PEU/PAA compared with 178°C for the PEU. When the heating rate was slowed to 10°C per minute, the Tₘ temperatures observed were 154°C for the PEU/PAA with 176 and 186°C for the PEU. In some analyses of the PEU, two Tₘ's were observed (176°C and 186°C), which may be due to different segments in the polymer. The change of the Tₘ is due at least in part to an increase in polymer volume caused by the addition of the PAA, leading to fewer hard segments per volume of polymer.
**Example 36** The coefficient of friction µ of a PEU/PAA semi-IPN of this disclosure against itself was measured real-time during a wear test using a built-in torque cell, and was found to range between 0.015 to 0.06, and as shown in Figure 42, is similar to cartilage-on-cartilage µ values, Because of its lower (compared to cartilage) permeability, the PEU/PAA semi-IPN of this disclosure can preserve a lower coefficient of friction for longer and at higher contact pressures. Figure 42 shows the effective coefficient of friction during a wear test of the joint interface material (labeled "PEU/PAA-on-PEU/PAA" in the graph) under 2.4 MPa of continuous (static) contact pressure. Literature reports on natural cartilage values and experimental data/literature reports on UHMWPE on CoCr are also presented in the plot (Mow, 2005; Wright 1982). As expected, the coefficient of friction was found to remain unchanged over the course of time when the load was applied in cycles of 1 Hz; similar results are reported for cartilage. The low coefficient of friction in the material can be explained in terms of (a) hydroplaning action, (b) load sharing between the solid and the fluid phases of the material (c) thin film lubrication as water persists on the surface of the material. The small increase of µ under static load can be explained by a small partial dehydration of the material under the pressure. In comparison, natural cartilage will lose most of its water under static load and therefore its coefficient of friction increases rapidly and to higher levels. Removal of the load and subsequent rehydration restores the initial coefficient of friction for natural cartilage.
**Example 37** The coefficient of friction is a number that indicates the force resisting lateral motion of an object. It is expressed as a unitless ratio of the frictional force to the normal force. The dynamic coefficient of friction for the polyether urethane/polyacrylic acid (PEU/PAA) semi-IPN on was tested on metal, and the dynamic coefficient of friction is shown as a function of time. Briefly, a piece of Elasthane™ 55D (polyetherurethane) was soaked in acrylic acid with an initiator and cross-linker, and cured to form a water swellable semi-IPN of the present disclosure. Plugs 8.8 mm in diameter and 1 mm thick were cut, swollen in PBS, and then rotated at a frequency of 1 Hz against a 3/16" stainless steel disc at a contact stress of 2.0 MPa while being submerged in PBS. Using a custom-made wear tester made according to ASTM F732 standards equipped with both a force load cell and a torque load cell, the dynamic coefficient of friction was measured real-time during the wear test experiment. The dynamic coefficient of friction of the material varied between 0.005 and 0.015 over a period of 36 hours.
**Example 38** Wear experiments of the PEU/PAA semi-IPN of this disclosure were conducted according to ASTM F732 using a pin-on-disc configuration. Results are shown in Figures 44, 45, and 45. Discs and pins formed from the joint interface material were tested to 2,500,000 cycles. As a basis for comparison to industry standard materials, a CoCr pin-on-UHMWPE (Cobalt chrome on ultra-high molecular weight polyethylene) disc configuration was also tested for 1,000,000 cycles.

In the test of the PEU/PAA semi IPN of this disclosure, the pins were 8.8 mm in diameter, 2.5 mm in thickness. The disc was 88 mm in diameter and 2.5 mm in thickness. The pins were rotated over the disc at a radius of 24 mm and at a rate of 1.33 Hz under a pneumatically applied cyclic load. A pressure regulator was used to adjust the air pressure so that the desired force was applied. The load was measured using a load cell (Sensotec Honeywell, CA) directly under the disc. The disc and the pins were mechanically isolated so that the torque caused by the friction generated between them can be measured by a torque cell (Transducer Techniques, CA) connected to a computer equipped with a data acquisition card (National Instruments, TX). The pin and discs were contained in a chamber filled with PBS. The temperature was controlled and kept constant at 37°C using a thermocouple-resistor-fan system. Using the equation µ = T/r*F, where T is the measured torque, r is the radius of rotation (= 24 mm) and F being the total force applied on the pins, the coefficient of friction was constantly monitored. The coefficient of friction was found to be 0.016 and independent of the contact pressure (range tested 0.1-3.5MPa) and slightly increased to 0.021 under heavy static contact load, but returned to the original value after fluid recovery. The wear was measured using the gravimetric method every million cycles: the disc and the pins were weighed separately after vacuum drying for 3 days. The wear test solution (PBS) was collected and visually examined; no signs of visible wear particles were noted at all steps of the tests. The wear test PBS solution was vacuum filtered using a 2.5 µm pore filter to capture any wear particles, flushed with deionized water to remove remaining PBS salts and then dried overnight under vacuum and desiccant. As a control, a similar test was performed using CoCr pins (Fort Wayne Metals, IN) on UHMWPE (Orthoplastics, UK). Three polished (Ra < 1.6µm) CoCr flat pins of OD = 7 mm were tested in the same instrument against a polished UHMWPE disc of 2.5mm thickness and OD = 88mm (rotation radius = 24mm), rotating at 1.2Hz under 3.4 MPa static contact load and at 37°C isolated environment.

Observation of the disc formed from the PEU/PAA semi-IPN of this disclosure after the test (Fig. 44A) revealed no macroscopically perceptible wear track along the pin-on-disc articulation surface. (Figure 44B is a close-up view of the location of the wear track. Dashed lines have been added to indicate the path; the radial arrows start from the center of the disc.) In comparison, as shown in Figure 44C, the UHMWPE disc after 2.0 M cycles of wear against CoCr pins has a visible track 126 µm deep.

Weighing of the wear test solution filtrate using a scale with a 0.01 mg resolution (Mettler Toledo, OH) showed that the volumetric wear rate of the PEU/PAA semi-IPN was approximately 0.6 mg/10⁶ cycles or 0.63 mm³/106 cycles or 0.63 mm³/150×10³ m. This value, however is close to the resolution of the methods. A schematic of the wear test solution from the wear test of the joint interface material comprised of PEU/PAA semi-IPN is shown in Figure 45A, demonstrating an absence of particles in the PBS solution. Compare Fig. 45A to schematics of the wear test solution of the UHMWPE disc shown in Figures 45B and 45C, which show substantial wear debris particles generated during the CoCr-on-UHMWPE wear test.

Although attention was paid to eliminate external factors such as dust, moisture and static in order to increase the accuracy of the results, the wear values are well near the statistical and practical detection limits of the methods available. These results are consistent with the hypothesis that since the PEU/PAA semi IPN according to the present disclosure— like natural cartilage—is comprised of mostly water, and the surface is persistently lubricated with a film of water, there is little, if any, contact between solid matrices.

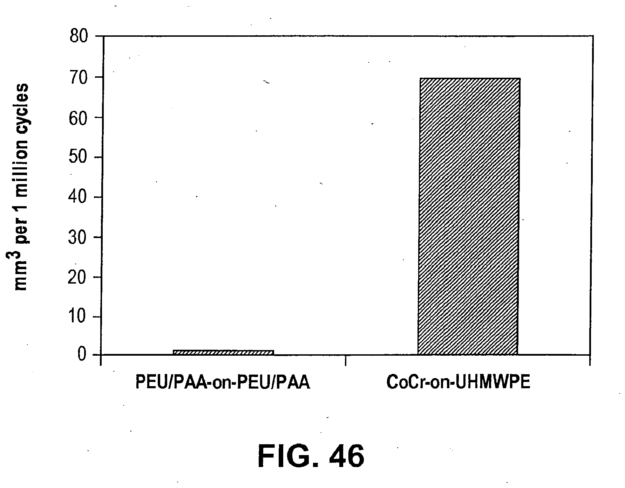

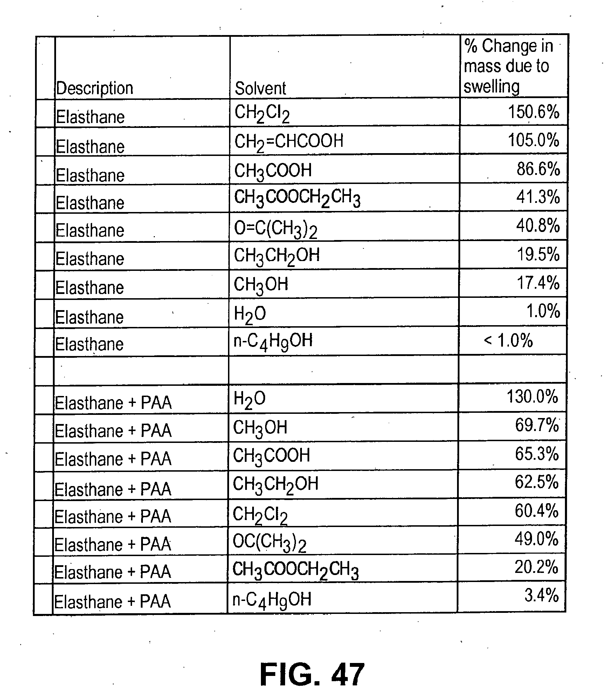

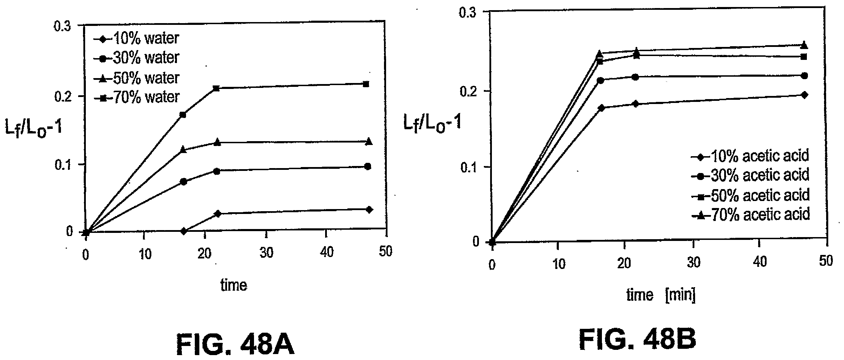

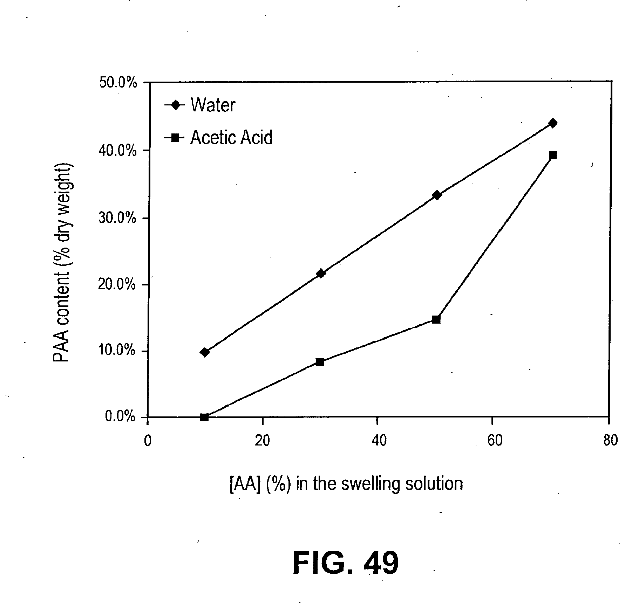

Wear particle measurements were also taken for the CoCr-on-UHMWPE experiments, which not only created a visible wear track (Fig. 44 B) on the UHMWPE disc, but generated substantial macroscopic wear debris (Fig. 45B and C). The UHMWPE disc was weighed and the difference in weight yielded an average wear rate of 64 mg/10⁶ cycles or 69 mm³/150×10³m (Figure 46). This study points that the joint interface material of this disclosure (labeled "PEU/PAA-on-PEU/PAA") is at least more than 100 more resistant to wear than the traditional combination of CoCr - UHMWPE, widely used in total joint replacements.
**Example 39** Figure 47 shows the swelling behavior of PEU/PAA and PEU in various aqueous and organic solvents. Briefly, a sheet of Elasthane™ 55D (polyether urethane) was soaked in acrylic acid with initiator and cross-linker, and cured to form a semi IPN. A small piece of the IPN or Elasthane™ 55D was obtained and weighed. The sample was soaked for 20 hours in a solution containing the solvent indicated in the Figure. (The samples were swollen, but did not dissolve). The sample was removed from the solvent, briefly dabbed dry, and then weighed again. The change in weight due to swelling is expressed as the % difference. While Elasthane™ 55D on its own does not take up water, the IPN of the present disclosure readily swells with water to form a lubricious, hydrated IPN. In addition, other solvents can be used to swell the starting polymer to create the IPN of the current disclosure. In the case of polyurethanes, the ability of various solvents to swell the material depends on the properties of the solvent (such as its polarity, acidity, and molecular weight) as well as the relative solubility of the polymer components (e.g. hard and soft segments) in the solvent.
**Example 40** The swelling of polyetherurethane by acrylic acid in water and acetic acid was tested. Swelling solutions were prepared containing 10, 30, 50, and 70 % acrylic acid monomer in deionized water (Figure 48A) and in acetic acid (Figure 48B). Small pieces of Elasthane™ ® 55D (polyetherurethane) were obtained and measured. A sample of the Elasthane™ was placed in each solution. The samples were removed from the solvent, the surface briefly dabbed dry, and then measured again. The change due to swelling is expressed as the final length of the specimen after equilibrium swelling (L_{f}) divided by the original length (Lₒ) minus 1; in this way, the fractional increase in length relative to the initial state (y = 0) is plotted versus time. Swelling of the Elasthane™ 55D was observed using either water or acetic acid as a solvent. More swelling was observed when a higher amount of acrylic acid was used in the swelling solution. Of note, the concentration dependence of acrylic acid on the swelling of the Elasthane™samples was different depending on whether water or acetic acid was used as the solvent.
**Example 41** Figure 49 shows the amount of poly(acrylic acid) present in the PEU/PAA semi-IPN after curing is plotted as a function of the starting concentration of acrylic acid monomer in different swelling solutions.

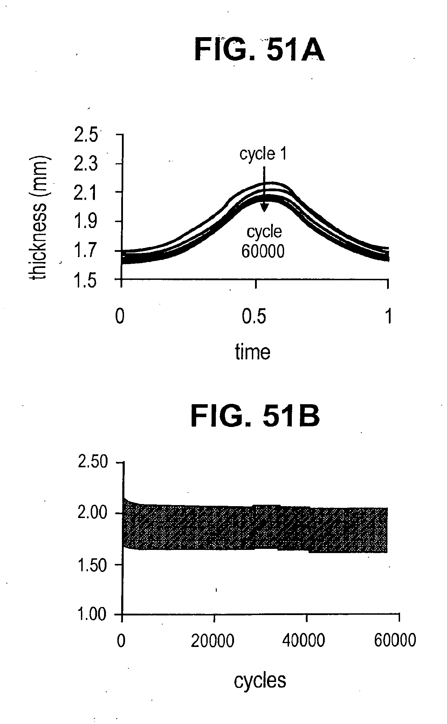

Swelling solutions were prepared containing 10, 30, 50, and 70 % acrylic acid monomer in deionized water and in acetic acid. Small pieces of Elasthane™ 55D (polyetherurethane) were obtained and weighed. Samples were placed in each of the water/acrylic acid or acetic acid/acrylic acid solutions along with cross-linker and initiator. The samples were cured, swollen in acrylic acid in either water or acetic acid, removed from the solution, dried, and then weighed again. Incorporation of acrylic acid into the Elasthane™ 55D to form a semi-IPN was observed using either water or acetic acid as solvent. More incorporation of acrylic acid was observed when a higher concentration of acrylic acid was present in the swelling solution.
**Example 42** Semi IPNs were prepared essentially as described in Figure 49, and the polyacrylic acid content of the IPNs was determined. The dried materials were weighed, swollen in saline until equilibrium was reached, and weighed again. The change in weight of the semi IPN is expressed as a ratio of the weight of the swollen material/weight of the dry material (Ws/Wd) for each concentration of polyacrylic acid. An increased amount of polyacrylic acid in the polymer correlates with an increased uptake of saline into the water-swellable semi-IPN. Since the semi-IPNs in these experiments were neutralized to pH 7.4, in these experiments, the dry weight of the semi-IPN included the salts present in the saline swelling solution, since the monovalent cations (predominantly sodium, which has a MW of 23 g/mol) are counterions to the carboxylate groups in the material.
**Example 43** Figures 51-54 show the results of creep and stress relaxation/compression testing. Tests were performed on PEU/PAA semi IPNs formed from Elasthane™ 55D (polyetherurethane) soaked in acrylic acid with initiator and cross-linker, and cured.

Figure 51 shows the results of cyclic compression testing. The behavior of the PEU/PAA semi IPN was tested under dynamic compression conditions to determine permanent creep and creep recovery. Permanent creep is the time-dependent deformation of a material under a constant load. Creep recovery measures the rate of decrease in the applied deformation after a load is removed. Experimental setup of the compression test followed the ASTM standard D695, Standard Test Method for Compressive Properties of Rigid Plastics, with the samples being subjected to a sinusoidal loading scheme designed to mimic the physiologic, cyclic compressive loads seen in a gait cycle.

A sample of the PEU/PAA semi IPN was removed and measured in the direction of its thickness, subject to cycles of compressive stress from 0-3 MPa at a frequency of 1 Hz for over 60,000 cycles, measured again in the direction of its thickness, re-equilibrated (relaxed) in PBS to allow for recovery from creep, and measured again in the direction of its thickness. Figure 51A shows the results of thickness measurements on representative samples subject to one-second long cycles of tests (at the 1st, 1000th, 10,000th, 20,000^{th}, 40,000^{th}, and 60,000^{th} cycles) superimposed in one figure. Figure 51B shows how the thickness of the material changes over all cycles of testing. The thickness of the material, as measured after load was removed during the cycle, dropped from an initial value of 2.160 mm at the first cycle to about 2.000 mm by the 60,000^{th} cycle. However, after re-equilibration (relaxation) in PBS and creep recovery at the last cycle, the material returned to a thickness of 2.135 mm, a total loss of thickness of only 1.1% due to permanent creep.

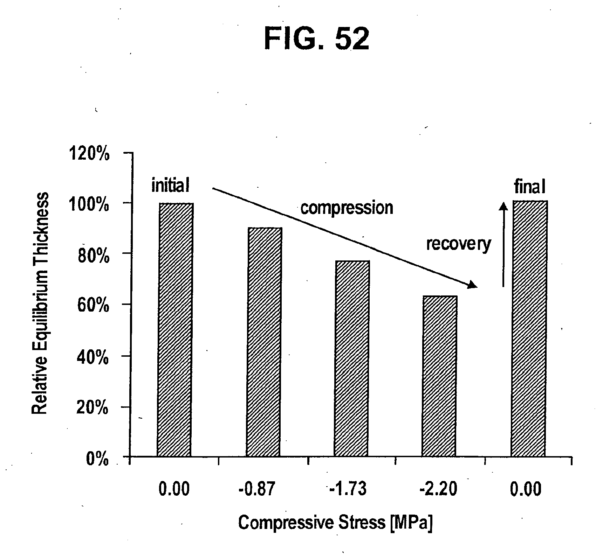

Figure 52 presents the equilibrium compressive behavior of the PEU/PAA semi IPN as determined through a multiple-step stress relaxation test, in which a given displacement is applied and then the material is allowed to relax (equilibrate). Notably, under these test conditions, the material fully recovered to its equilibrium value after removal of the load, as shown by the last data point in the figure 52, indicating full creep recovery. The stress of 2.20 MPa (4th data point) is 15% higher than the maximum functional stress in a hip device (total load through the hip of 3 times body weight) that is predicted by finite element models.

A static creep test was also performed (data not shown). Creep is the time-dependent deformation of a material under a constant load. The behavior of the PEU/PAA semi IPN tested under static compression was tested following ASTM D2290-01 "Standard Test Methods for Tensile, Compressive, and Flexural Creep and Creep-Rupture of Plastics". A plug of the PEU/PAA semi IPN with an initial diameter of 9.525 mm and a thickness of 1.115 mm was put under an initial stress 4 MPa in a fluid PBS bath. After applying the stress for approximately 20,000 seconds (to a total strain of 14.29%), the load was released and the material allowed to relax (re-equilibrate) in PBS. The final thickness of the plug was 1.109 mm. The final unrecovered creep after more than 40,000 cycles was 2.7 %.

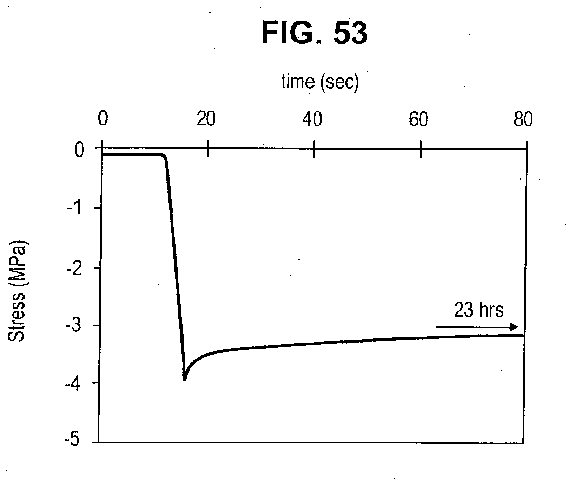

Figure 53 shows the results of a compression set test according to ASTM D395. In this test, a plug of PEU/PAA with an initial diameter of 9.525 mm and a thickness of 2.13 mm was subjected to a constant compressive strain of 15% for 23 hours at room temperature in a fluid bath filled with PBS. After allowing the material to relax and re-equilibrate in PBS, the final thickness of the plug was 2.08 mm. This yields a compression set value of 9.5%. As a basis of comparison, PEU (Elasthane™55D) alone exhibits a compression set value of about 45% under the same conditions (22 hrs, room temperature). Therefore, the presence of the polyelectrolyte in the PEU/PAA semi-IPN provides a way for the PEU material to resist permanent creep through rehydration of the matrix with water due to the hydrophilicity and high swellability of the negatively charged polyelectrolyte.

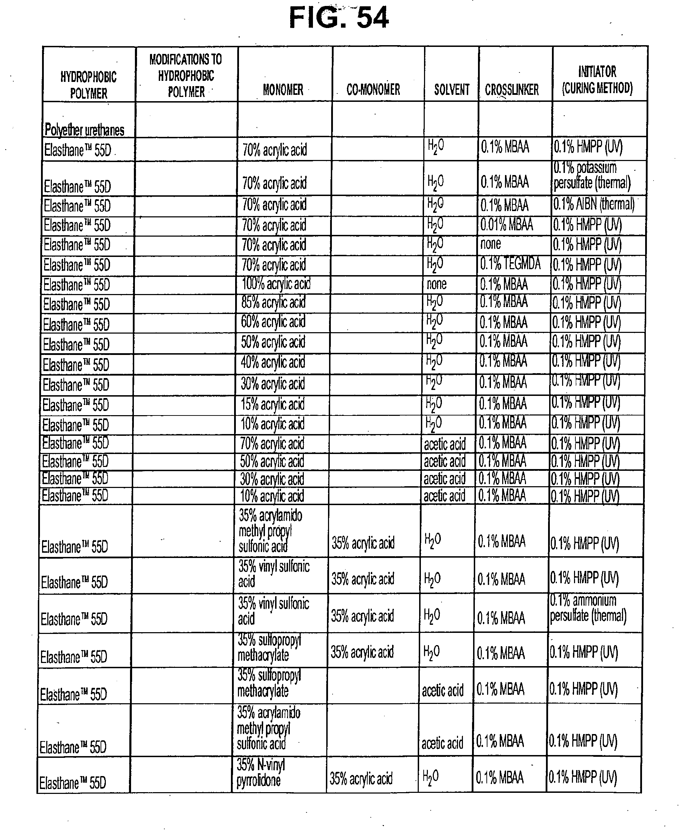

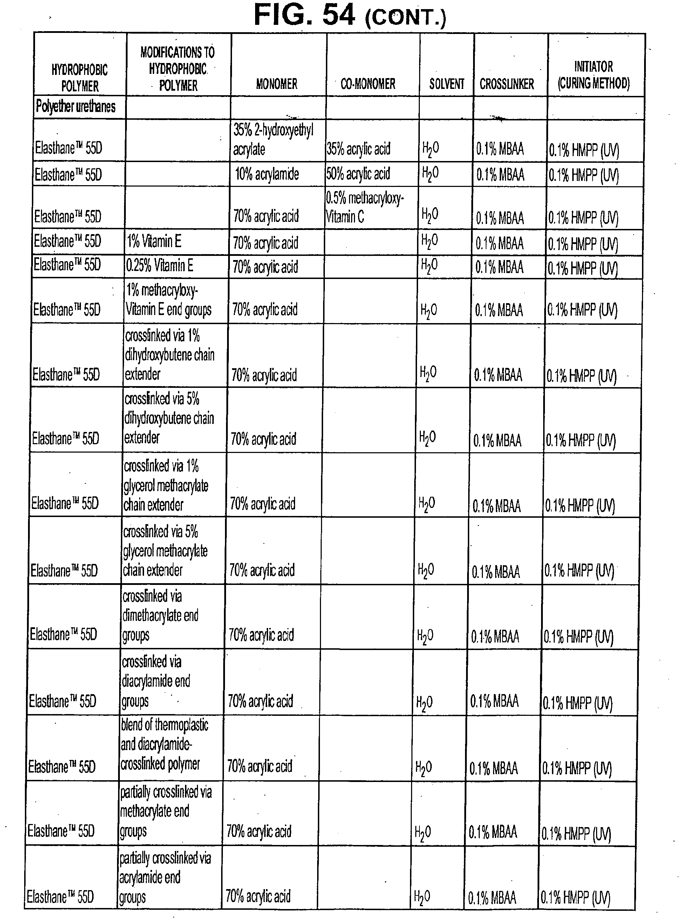

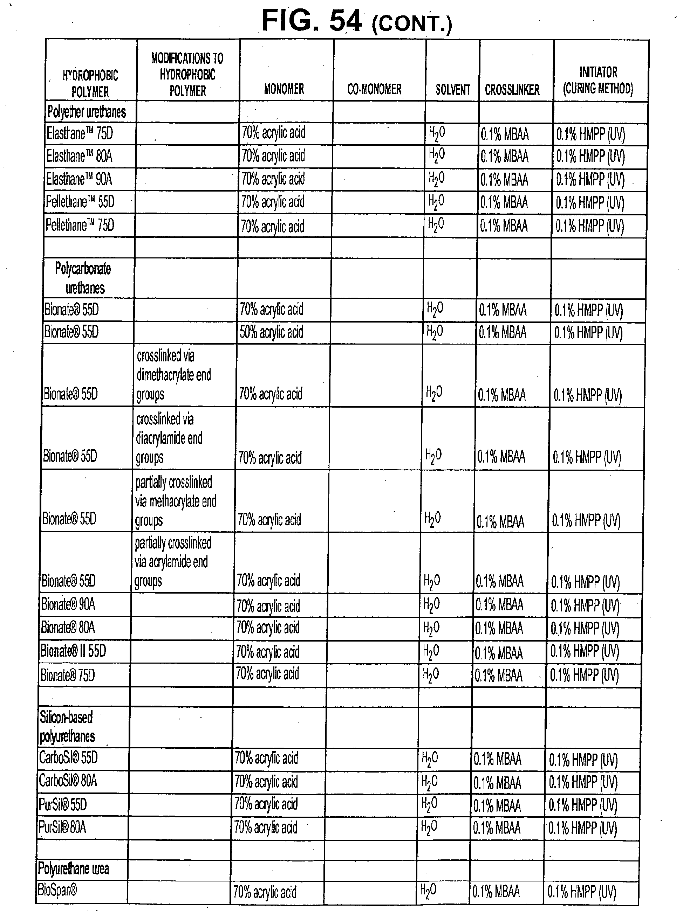

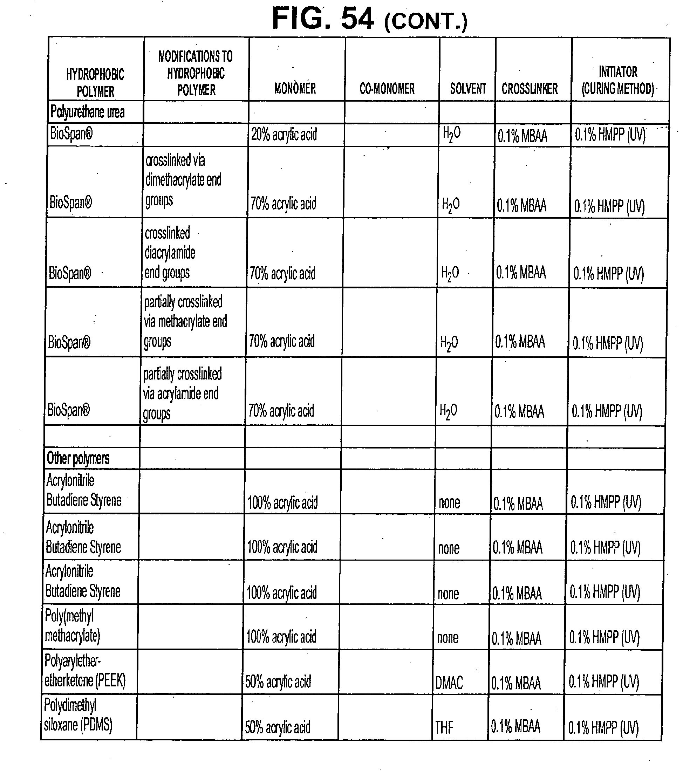

**Example 44** Figure 54 shows a list of some of the materials made in accordance with the present disclosure. The first column shows the hydrophobic polymer used. If a modification was made to the hydrophobic polymer as indicated in the second column, the material for the modification was cast with the material, or, if the modification was crosslinking functionality, the modification was added and the material prepared and crosslinked and used thereafter with the crosslinks reacted. The monomer, comonomer (if any), crosslinker and initiator were added in the indicated solvent as indicated in the figure in order to swell the prepared hydrophobic polymer. Each hydrophobic polymer sample was allowed to swell for up to 2 days, removed from the solution, and cured using the indicated method following standard procedures. The material was washed and swollen in PBS. The abbreviations used are as follows: MBAA = methylene bisacrylamide, HMPP = 2-hydroxy-2-methyl propiophenone, TEGDMA = triethylene glycol dimethacrylate, and H₂0 = water.

## Claims

1. A composition of matter comprising a negatively charged water-swellable IPN or semi-IPN comprising a hydrophobic thermoset or thermoplastic polyurethane polymer and an ionic polymer comprising sulfonate groups physically entangled with the polyurethane, wherein the ionic polymer forms a concentration gradient from a first portion of the composition to a second portion of the composition.

2. The composition of claim 1 wherein the IPN or semi-IPN exhibits a lower coefficient of friction than the hydrophobic thermoset or thermoplastic polymer.

3. The composition of claim 1 wherein the IPN or semi-IPN is more water-swellable than the hydrophobic thermoset or thermoplastic polymer.

4. The composition of claim 1 wherein the IPN or semi-IPN exhibits a higher conductivity than the hydrophobic thermoset or thermoplastic polymer.

5. The composition of claim 1 wherein the IPN or semi-IPN is formed by diffusing an ionizable monomer precursor into the hydrophobic thermoset or thermoplastic polymer and polymerizing the monomer to form the ionic polymer.

6. The composition of claim 1 further comprising water.

7. The composition of claim 6 wherein the water forms a hydration gradient from a first portion of the composition to a second portion of the composition.

8. The composition of claim 1 wherein the ionic polymer comprises carboxylate groups and sulfonate groups.

9. The composition of claim 1 wherein the concentration gradient provides a stiffness gradient within the composition.

10. The composition of claim 1 wherein the hydrophobic thermoset or thermoplastic polymer comprises a first hydrophobic thermoset or thermoplastic polymer, the composition further comprising a second hydrophobic thermoset or thermoplastic polymer.

11. The composition of claim 10 wherein the second hydrophobic thermoset or thermoplastic polymer is disposed in a layer separate from the first hydrophobic thermoset or thermoplastic polymer.

12. The composition of claim 10 wherein the second hydrophobic thermoset or thermoplastic polymer is diffused throughout the first hydrophobic thermoset or thermoplastic polymer.

13. The composition of claim 1 wherein the ionic polymer comprising sulfonate groups is a polymer comprising 2-acrylamido-2-methylpropanesulfonic acid monomers.

14. The composition of claim 13 wherein the ionic polymer is a copolymer of acrylic acid and 2-acrylamido-2-methylpropanesulfonic acid.

## Patentansprüche

1. Stoffzusammensetzung, die Folgendes umfasst: ein negativ geladenes wasserquellbares IPN oder semi-IPN , das ein hydrophobes duroplastisches oder thermoplastisches Polyurethanpolymer umfasst, und ein ionisches Polymer, das Sulfonatgruppen umfasst, die physikalisch mit dem Polyurethan verschränkt sind, wobei das ionische Polymer einen Konzentrationsgradienten von einem ersten Teil der Zusammensetzung zu einem zweiten Teil der Zusammensetzung bildet.

2. Zusammensetzung nach Anspruch 1, wobei das IPN oder Semi-IPN einen niedrigeren Reibungskoeffizienten aufweist als das hydrophobe duroplastische oder thermoplastische Polymer.

3. Zusammensetzung nach Anspruch 1, wobei das IPN oder Semi-IPN stärker wasserquellbar ist als das hydrophobe duroplastische oder thermoplastische Polymer.

4. Zusammensetzung nach Anspruch 1, wobei das IPN oder Semi-IPN eine höhere Leitfähigkeit aufweist als das hydrophobe duroplastische oder thermoplastische Polymer.

5. Zusammensetzung nach Anspruch 1, wobei das IPN oder Semi-IPN gebildet wird, indem ein ionisierbarer Monomervorläufer in das hydrophobe duroplastische oder thermoplastische Polymer diffundiert und das Monomer polymerisiert wird, um das ionische Polymer zu bilden.

6. Zusammensetzung nach Anspruch 1, die ferner Wasser umfasst.

7. Zusammensetzung nach Anspruch 6, wobei das Wasser einen Hydratationsgradienten von einem ersten Teil der Zusammensetzung zu einem zweiten Teil der Zusammensetzung bildet.

8. Zusammensetzung nach Anspruch 1, wobei das ionische Polymer Carboxylatgruppen und Sulfonatgruppen umfasst.

9. Zusammensetzung nach Anspruch 1, wobei der Konzentrationsgradient einen Steifigkeitsgradienten innerhalb der Zusammensetzung bereitstellt.

10. Zusammensetzung nach Anspruch 1, wobei das hydrophobe duroplastische oder thermoplastische Polymer ein erstes hydrophobes duroplastisches oder thermoplastisches Polymer umfasst, wobei die Zusammensetzung ferner ein zweites hydrophobes duroplastisches oder thermoplastisches Polymer umfasst.

11. Zusammensetzung nach Anspruch 10, wobei das zweite hydrophobe duroplastische oder thermoplastische Polymer in einer Schicht getrennt von dem ersten hydrophoben duroplastischen oder thermoplastischen Polymer angeordnet ist.

12. Zusammensetzung nach Anspruch 10, wobei das zweite hydrophobe duroplastische oder thermoplastische Polymer durch das erste hydrophobe duroplastische oder thermoplastische Polymer diffundiert ist.

13. Zusammensetzung nach Anspruch 1, wobei das ionische Polymer, das Sulfonatgruppen umfasst, ein Polymer mit 2-Acrylamido-2-methylpropansulfonsäure-Monomeren ist.

14. Zusammensetzung nach Anspruch 13, wobei das ionische Polymer ein Copolymer von Acrylsäure und 2-Acrylamido-2-methylpropansulfonsäure ist.

## Revendications

1. Composition de matière comprenant un IPN ou semi-IPN gonflable à l'eau chargé négativement comprenant un polymère polyuréthane thermodurcissable ou thermoplastique hydrophobe et un polymère ionique comprenant des groupes sulfonate physiquement enchevêtrés avec le polyuréthane, dans laquelle le polymère ionique forme un gradient de concentration à partir d'une première partie de la composition à une deuxième partie de la composition.

2. Composition selon la revendication 1 dans laquelle l'IPN ou le semi-IPN présente un coefficient de frottement inférieur à celui du polymère thermodurcissable ou thermoplastique hydrophobe.

3. Composition selon la revendication 1, dans laquelle l'IPN ou le semi-IPN est plus gonflable à l'eau que le polymère thermodurcissable ou thermoplastique hydrophobe.

4. Composition selon la revendication 1, dans laquelle l'IPN ou le semi-IPN présente une conductivité plus élevée que le polymère thermodurcissable ou thermoplastique hydrophobe.

5. Composition selon la revendication 1, dans laquelle l'IPN ou le semi-IPN est formé en diffusant un précurseur de monomère ionisable dans le polymère thermodurcissable ou thermoplastique hydrophobe et en polymérisant le monomère pour former le polymère ionique.

6. Composition selon la revendication 1, comprenant en outre de l'eau.

7. Composition selon la revendication 6, dans laquelle l'eau forme un gradient d'hydratation à partir d'une première partie de la composition à une deuxième partie de la composition.

8. Composition selon la revendication 1, dans laquelle le polymère ionique comprend des groupes carboxylate et des groupes sulfonate.

9. Composition selon la revendication 1, dans laquelle le gradient de concentration fournit un gradient de rigidité dans la composition.

10. Composition selon la revendication 1, dans laquelle le polymère thermodurcissable ou thermoplastique hydrophobe comprend un premier polymère thermodurcissable ou thermoplastique hydrophobe, la composition comprenant en outre un deuxième polymère thermodurcissable ou thermoplastique hydrophobe.

11. Composition selon la revendication 10 dans laquelle le deuxième polymère thermodurcissable ou thermoplastique hydrophobe est disposé dans une couche séparée du premier polymère thermodurcissable ou thermoplastique hydrophobe.

12. Composition selon la revendication 10, dans laquelle le deuxième polymère thermodurcissable ou thermoplastique hydrophobe est diffusé dans tout le premier polymère thermodurcissable ou thermoplastique hydrophobe.

13. Composition selon la revendication 1, dans laquelle le polymère ionique comprenant des groupes sulfonate est un polymère comprenant des monomères d'acide 2-acrylamido-2-méthylpropanesulfonique.

14. Composition selon la revendication 13, dans laquelle le polymère ionique est un copolymère d'acide acrylique et d'acide 2-acrylamido-2-méthylpropanesulfonique.
